# EUROPEAN PATENT APPLICATION

(11) **EP 2 233 485 A1**
(43) Date of publication of application: **29.09.2010**
(21) Application number: 09155910.4
(22) Date of filing: 23.03.2009
(51) Int. Cl.: C07D 413/14, C07D 493/10, C07F 3/00, G01N 33/58

(54) **Fluorophore-containing compounds and their use in the detection of phosphorylated (poly)peptides**

(71) Applicant: Universität Regensburg, 93053 Regensburg (DE)
(72) Inventor: Schmidt, Florian, 93142 Maxhütte-Haidhof (DE); König, Burkhard, 93138 Lappersdorf (DE); Stadlbauer, Stefan, 93449 Waldmünchen (DE); Riechers, Alexander, 83547 Babensham (DE)
(74) Representative: Vossius & Partner

(57) **Abstract**

The present invention relates to certain fluorophor compounds and a method of detecting a phosphorylated peptide or phosphorylated polypeptide using the same. Furthermore, a method of detecting and differentiating phosphorylated and unphosphorylated peptides or polypeptides is described. A kit and a diagnostic composition comprising the fluorophor compounds as well as a method of diagnosing a disease are also disclosed.

## Description

### Field of the invention

The present invention relates to certain fluorophor compounds and a method of detecting a phosphorylated peptide or phosphorylated polypeptide using the same. Furthermore, a method of detecting and differentiating phosphorylated and unphosphorylated peptides or polypeptides using the fluorophor compounds is described. A kit and a diagnostic composition comprising the fluorophor compounds as well as a method of diagnosing a disease are also disclosed.

### Background of the invention

Detection and quantitation of proteins are standard techniques in molecular biology. They may be performed in solution or on supports, supports including gels, membranes and filters. As regards protein detection on supports, in particular on gels, this process is often referred to as "staining".

While silver- and Coomassie-staining are widely used for total protein staining, a number of stains selective for certain functional groups have emerged. Glycosylation (1, 2), His-tags (3) and phosphorylation (4, 5) are typical protein modifications targeted by selective gel stains reported so far.

With respect to its biological importance, phosphorylation (6, 7) is widely regarded as the most significant post-translational modification. Phosphorylation plays an important role in signaling pathways and it is estimated that 30 % of the entire proteome becomes phosphorylated at some point (8). While there are phospho-specific antibodies available (9, 10), they usually are specific for epitopes comprising the proximity of the phosphorylation site. As a consequence, not every phosphorylated site in a protein is amenable to detection with a given antibody. Alternatively, ³²P-labeling of the proteins provides a very sensitive tool for detection of phosphorylation (11, 12). However, the handling and disposal of radioactive material are costly, potentially hazardous and increasingly regulated. Therefore, when staining for phosphorylation, fluorescence detection is the method of choice due to its inherent sensitivity. The reported (13) and commercially available (4, 5) fluorescent phospho-specific stains achieve their sensitivity from their binding site specificity. While other fluorescent probes for phosphorylated amino acids have been reported (14), their selectivity has only been demonstrated for peptides without other metal-chelating amino acids like histidine, tryptophan or cysteine.

Grauer (15) describes organic molecules designated as "synthetic receptors" which differentiate between different phosphorylated peptides. The synthetic receptors comprise a bis-Zn^{II}-cyclen complex. Also described are synthetic receptors which are conjugated to further moiety designed to bind to an amino acid in position (i+3) relative to the phosphorylated position. A fluorophore is not described in Grauer (15).

Mizukami (16) describes fluorescent anion sensors. The described sensor molecules comprise a mono-metal-cylen complex conjugated to a fluorophore. Cd" is considered the most suitable metal ion, whereas the Zn" complex is described as being unable to act as an ion sensor. The detected anions include pyrophosphate and nucleotides such as AMP and cAMP.

In view of the prior art, the technical problem can be considered as the provision of alternative or improved means and methods for detecting phosphorylated peptides and polypeptides.

### Summary of the invention

The main aspects of the invention are summarized in the following items:
1. A compound comprising an anion and a cation having the general formula (I): 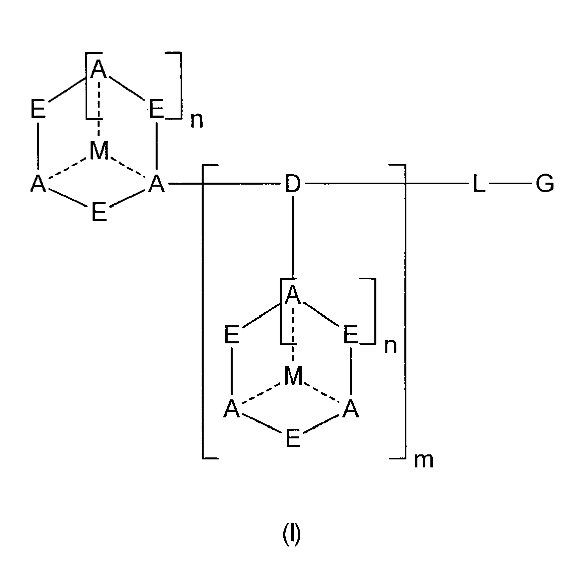 wherein:
   A is selected from the group consisting of S, O, NH and PH;
   E is selected from the group consisting of CH₂ and CH₂CH₂;
   M is selected from the group consisting of Zn, Mn, Fe, Sc, Ti, Zr, Eu, Gd, and Tb;
   D is a linking group;
   L is a linking group;
   G is a substituted or unsubstituted fluorophor group of the general formula (II), (III) or (IV): 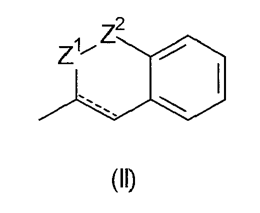 wherein
      Z¹ is selected from -CH₂- and -C(O)-;
      Z² is selected from -CH₂- and -O-; and ----- is a single or double bond; 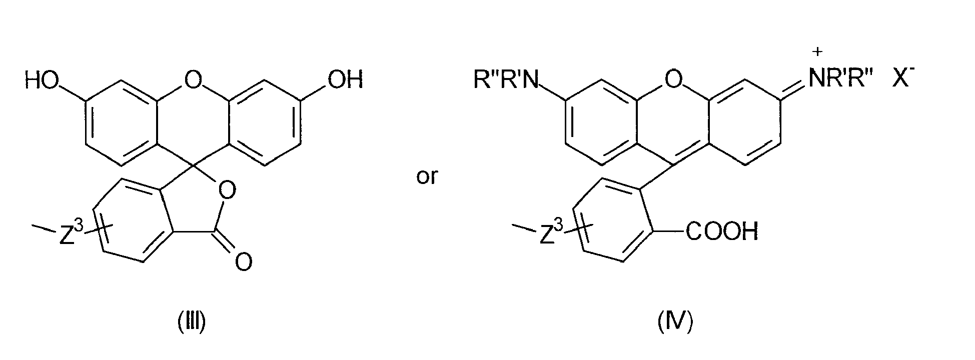 wherein
      Z³ is -CH₂-, -C(O)- or -C(S)-;
      R' and R" are independently selected for each occurrence and are selected from the group consisting of H and C₁₋₄ alkyl; and
      X is an anion;
      n is an integer from 1 to 6;
      m is 0 or 1, with the proviso that m = 1 if the fluorophor group is substituted or unsubstituted coumarin; and
      "-" indicates a chemical bond, while "-----" indicates that the metal M is coordinated by the moiety A.
2. A method of detecting a phosphorylated peptide or phosphorylated polypeptide, the method comprising:
   (a) bringing into contact said phosphorylated peptide or phosphorylated polypeptide with a compound as defined in item 1; and
   (b) detecting a complex comprising said phosphorylated peptide or phosphorylated polypeptide and said compound, thereby detecting said phosphorylated peptide or phosphorylated polypeptide.
3. A method of detecting a phosphorylated peptide or phosphorylated polypeptide, the method comprising:
   (a) bringing into contact said phosphorylated peptide or phosphorylated polypeptide with a compound comprising an anion and a cation having the general formula (I): 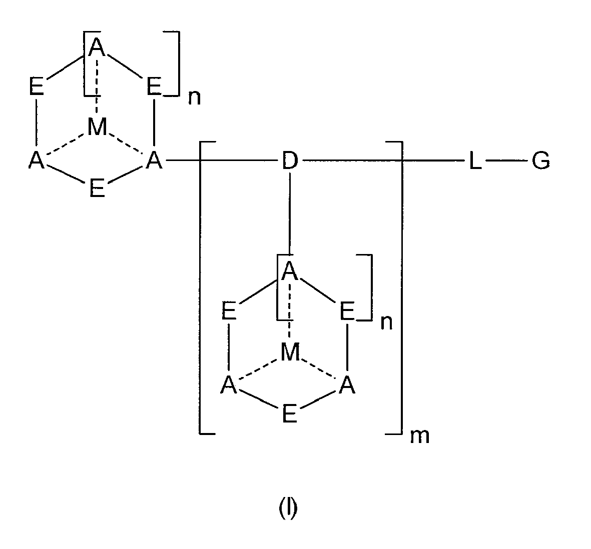 wherein:
      A is selected from the group consisting of S, O, NH and PH;
      E is selected from the group consisting of CH₂ and CH₂CH₂;
      M is selected from the group consisting of Zn, Mn, Fe, Sc, Ti, Zr, Eu, Gd, and Tb;
      D is a linking group;
      L is a linking group;
      G is a substituted or unsubstituted fluorophor group of the general formula (II), (III) or (IV): 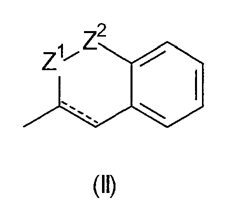 wherein
         Z¹ is selected from -CH₂- and -C(O)-;
         Z² is selected from -CH₂- and -O-; and
         ----- is a single or double bond; 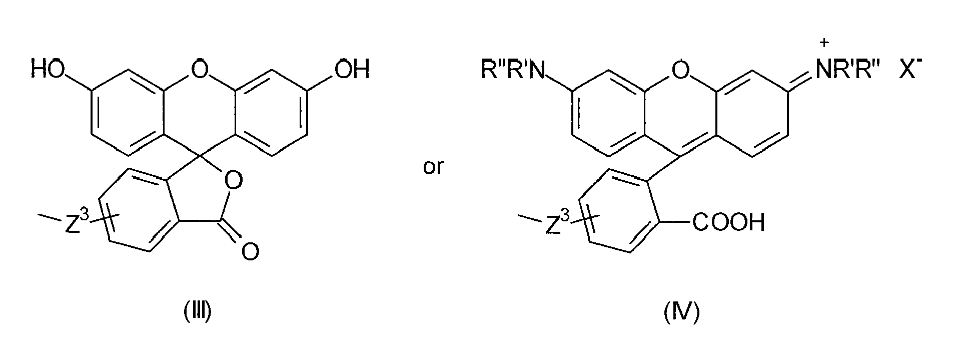 wherein
         Z³ is -CH₂-, -C(O)- or -C(S)-;
         R' and R" are independently selected for each occurrence and are selected from the group consisting of H and C₁₋₄ alkyl; and
         X is an anion;
         n is an integer from 1 to 6;
         m is 0 or 1; and
         "-" indicates a chemical bond, while "-----" indicates that the metal M is coordinated by the moiety A; and
   (b) detecting a complex comprising said phosphorylated peptide or phosphorylated polypeptide and said compound, thereby detecting said phosphorylated peptide or phosphorylated polypeptide;
      wherein the method is effected on or in a support.
4. A method of detecting and differentiating phosphorylated and unphosphorylated peptides or polypeptides, the method comprising:
   (a) bringing into contact said peptides or polypeptides with a compound as defined in item 1; and
   (b) determining
      (i) whether fluorescence emission being characteristic of a complex of said compound with phosphorylated peptides or phosphorylated polypeptides occurs; and
      (ii) whether fluorescence emission being characteristic of a complex of said compound with unphosphorylated peptides or unphosphorylated polypeptides occurs,
         thereby detecting and differentiating phosphorylated and unphosphorylated peptides or polypeptides.
5. A method of detecting and differentiating phosphorylated and unphosphorylated peptides or polypeptides, the method comprising:
   (a) bringing into contact said peptides or polypeptides with a compound as defined in claim 3; and
   (b) determining
      (i) whether fluorescence emission being characteristic of a complex of said compound with phosphorylated peptides or phosphorylated polypeptides occurs; and
      (ii) whether fluorescence emission being characteristic of a complex of said
         compound with unphosphorylated peptides or unphosphorylated
         polypeptides occurs,
         thereby detecting and differentiating phosphorylated and unphosphorylated peptides or polypeptides;
         wherein the method is effected on or in a support.
6. Use of a compound as defined in item 1 for detecting a phosphorylated peptide or phosphorylated polypeptide.
7. Use of a compound as defined in item 3 for detecting a phosphorylated peptide or phosphorylated polypeptide, wherein the detection is effected on or in a support.
8. Use of a compound as defined in item 1 for detecting and differentiating phosphorylated and unphosphorylated forms of a peptide or polypeptide.
9. Use of a compound as defined in item 3 for detecting and differentiating phosphorylated and unphosphorylated forms of a peptide or polypeptide, wherein the detection is effected on or in a support.
10. A kit comprising one or more compounds as defined in item 1.
11. A diagnostic composition comprising one or more compounds as defined in item 1.
12. A method of diagnosing a disease characterized by the presence of an aberrant amount of a phosphorylated peptide or phosphorylated polypeptide, the method comprising:
   (a) bringing into contact a sample obtained from a subject suspected to suffer from said disease with a compound as defined in item 1 or 3; and
   (b) determining whether the fluorescence emission of said sample differs from the fluorescence emission of a control sample.

Preferably the method of diagnosing a disease is effected on or in a support.

### Brief description of the figures

- **Figure 1:**: A scheme illustrating the discrimination between phosphorylated and non- phosphorylated proteins on a solid support.
- **Figure 2:**: Gel stained with probe **1**. Each lane contains 1 µg BSA (66 kDa). From left to right: lane 1: 1 µg α-casein (23 kDa) dephosphorylated, lanes 2 - 8: 1 µg, 500 ng, 250 ng, 125 ng, 62 ng, 31 ng, 15 ng α-casein. The top image was taken on a UV table (λₑₓ = 316 nm), the lower image shows CBB R-250 total protein restain.
- **Figure 3:**: Gel stained with probe **2**. Each lane contains 1 µg BSA (66 kDa). From left to right: lane 1: 1 µg α-casein (23 kDa) dephosphorylated, lanes 2 - 8: 1 µg, 500 ng, 250 ng, 125 ng, 62 ng, 31 ng, 15 ng α-casein. The top image was taken on a UV table (λₑₓ = 316 nm), lower image shows CBB R-250 total protein restain.
- **Figure 4:**: Normalized emission spectra of gel bands stained with probe **2** acquired through a 455 nm longpass filter (λₑₓ = 316 nm). The BSA band showed the same spectrum as dephosphorylated α-casein (data not shown).

### Detailed description of the invention

The fluorophor compounds of the present invention comprise an anion and a cation having the general formula (I): 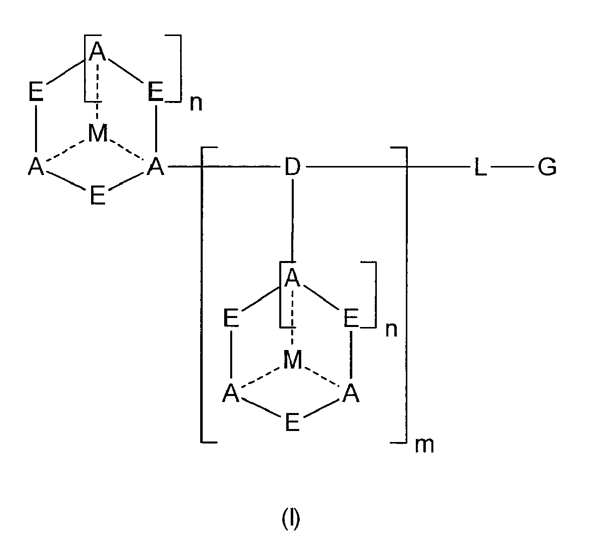 wherein
A is selected from the group consisting of S, O, NH and PH;
E is selected from the group consisting of CH₂ and CH₂CH₂;
M is selected from the group consisting of Zn, Mn, Fe, Sc, Ti, Zr, Eu, Gd, and Tb;
D is a linking group;
L is a linking group;
G is a substituted or unsubstituted fluorophor group of the general formula (II), (III) or (IV): 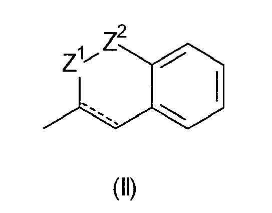 wherein
Z¹ is selected from -CH₂- and -C(O)-;
Z² is selected from -CH₂- and -O-; and
----- is a single or double bond; 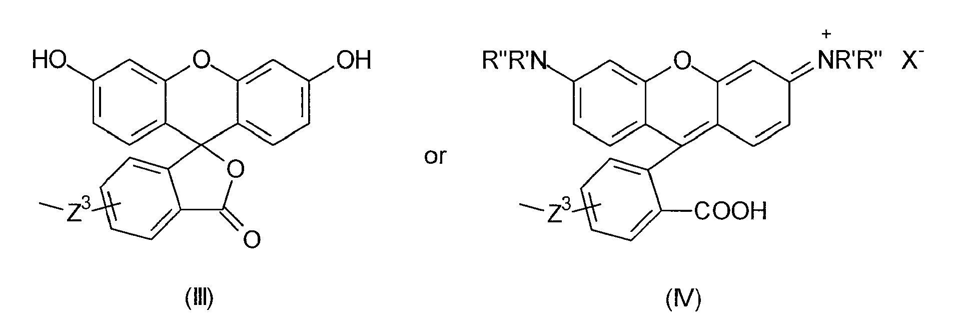 wherein
Z³ is -CH₂-, -C(O)- or -C(S)-;
R' and R" are independently selected for each occurrence and are selected from the
group consisting of H and C₁₋₄ alkyl; and
X is an anion; and
n is an integer from 1 to 6.

In some embodiments (among others, if e.g., the methods are effected in solution), m is 0 or 1, with the proviso that m = 1 if the fluorophor group is substituted or unsubstituted coumarin. The coumarin group has the formula:

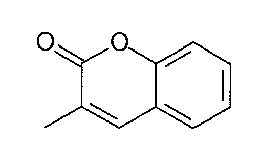

In those embodiments in which the methods are effected on or in a support, m is 0 or 1.

In the above formula (I) "-" indicates a chemical bond, while "-----" indicates that the metal M is coordinated by the moiety A.

The anion is not specifically limited. A skilled person can chose one or more anions, so that the compound of the present invention has a neutral charge based on the charge of the metal M. Examples of suitable anions are neutral anions such as chloride, perchlorate, and nitrate. Preferred anions are chloride and perchlorate.

The moiety A serves to complex or coordinate the metal atom M in the crown ether or its N-, S- or P-analogue. It is selected from the group consisting of S, O, NH and PH, preferably from the group consisting of O and NH: Most preferably A is NH because of its Lewis basicity and its ability to complex the "hard" metal cation.

E is selected from the group consisting of CH₂ and CH₂CH₂ and is preferably CH₂CH₂.

M is selected from the group consisting of Zn, Mn, Fe, Sc, Ti, Zr, Eu, Gd, and Tb. In a preferred embodiment M is selected from the group consisting of Zn and Mn, more preferably M is Zn. The selected metals, especially those of the preferred embodiments, not only have a high bindung affinity but also very low non-specific interactions.

D is a linking group. The nature of the linking group is not particularly limited and it can be chosen from any linking group which does not interfer with the detection of phosphorylation. Examples of the linking group include 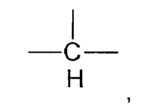 a 5- to 7-membered carbocyclic group and a 5- to 7-membered heterocyclic group containing one or more heteroatoms selected from N, P, O and S. The carbocyclic group and heterocyclic group can be saturated or include one or more double bonds.

Examples of carbocyclic groups include cyclopentyl, cyclohexyl, cycloheptyl and phenyl.

Examples of the heterocyclic group include

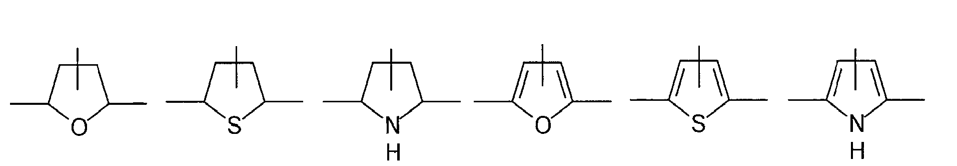

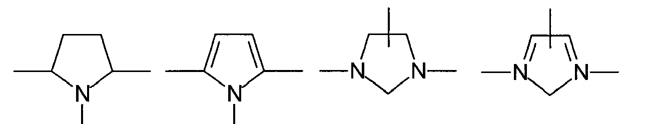

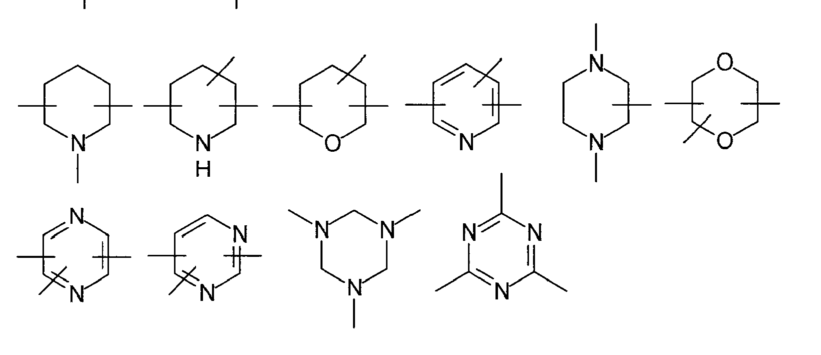

Preferably D is selected from 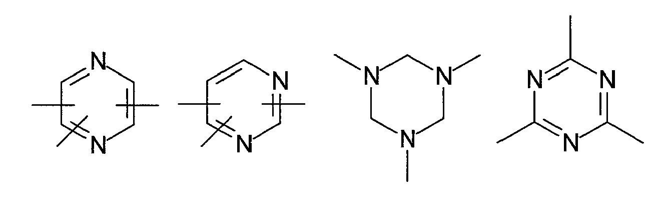 Most preferably D is 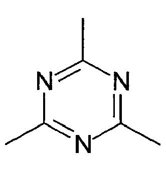 because its inflexible aromatic system increases the binding affinity.

n is an integer from 1 to 6, preferably n is 2.

m is as defined above. In a preferred embodiment m is 1 because the binding affinity to phosphate is higher. Therefore, the influence of the metal complex on the fluorophor is increased which in turn results in increased fluorescence when the phosphate group is bound.

L is a linking group. The nature of the linking group is not particularly limited and it can be chosen from any linking group which does not interfer with the detection of phosphorylation.

Examples of the linking group include -Xₚ-(CH₂)_{q}-Yᵣ- in which
X is selected from -O-, -S- and -NH-, preferably X is -NH-.

p = 0 or 1, preferably p = 1.

q = 1 to 6, preferably q = 2 to 4. The short chain length promotes the influence of the phosphate coordination on the fluoresence.

Y is selected from -O-, -S-, -NH-, -C(O)-, -SO₂NH-, -NHSO₂- -C(O)NH-, -NHC(O)-, -C(O)O- and -OC(O)-. Preferably Y is selected from -SO₂NH-, -NHSO₂-, -C(O)NH-, and -NHC(O)-, more preferably Y is selected from -C(O)NH-, and -NHC(O)-. The linking group is particularly stable and easy to synthesize in the preferred embodiments.

r is 0 or 1, preferably r is 1.

Mizukami (16) described a fluorescent anion sensor which functioned in neutral aqueous solutions. In such solutions a heteroatom which could coordinate to the metal had to be present in the linker between the cyclen and the fluorescent group otherwise the complexes were found to be unsuitable as an anion sensor. The present inventors have found that such a heteroatom is not necessary in the methods of the present invention. Consequently the selection of the linker is less restricted and metal chelates which have a high binding affinity with respect to the phosphate bond can be selected.

The alkylene chain in the group -Xₚ-(CH₂)_{q}-Yᵣ- can optionally be interrupted by 1 or 2 -O-, -S- or -NH- groups with the proviso that the -O-, -S- and -NH- groups, if present, are not directly adjacent to each other or the other heteroatom-containing groups X and Y, if present.

Preferred groups -Xₚ-(CH₂)_{q}-Yᵣ- are -NH-(CH₂)_{q}-NHC(O)- and -O-(CH₂)_{q}-O- where q is as defined above. These groups result ensure that the resultant compounds have a preferable solubility and stability.

A more preferred linker group is -NH-(CH₂)_{q}-NHC(O)- with q = 2 to 4.

G is a substituted or unsubstituted fluorophor group. Fluorophor groups within the meaning of the present invention are fluorophor groups of the general formula (II), (III) or (IV).

In one embodiment the fluorophor compound has the general formula (II): 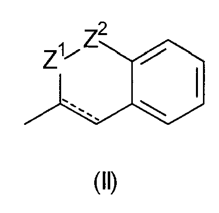 wherein
Z¹ is selected from -CH₂- and -C(O)-, and is preferably -C(O)-;
Z² is selected from -CH₂- and -O-, and preferably is -O-; and
----- is a single or double bond, preferably a double bond.

Of the groups having the formula (II) substituted and unsubstituted coumarin groups are preferred.

The groups having the formula (II) can be substituted in any available position by one or more substituents. The substituents are not particularly limited as long as they do not interfer with the detection of phosphorylation. Possible substituents include C₁₋₄ alkyl groups, OH, CF₃, NH₂, NH(C₁₋₄ alkyl), N(C₁₋₄ alkyl)₂, NO₂, Hal, SO₃H and phenyl. Preferred substituents are N(C₁₋₄ alkyl)₂, OH, NO₂ and SO₃H, more preferred substituents are N(C₁₋₄ alkyl)₂.

Preferred examples of groups having the formula (II) are 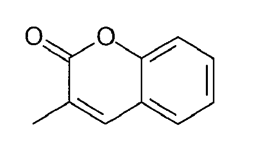 which are substituted by N(C₁₋₄ alkyl)₂, especially

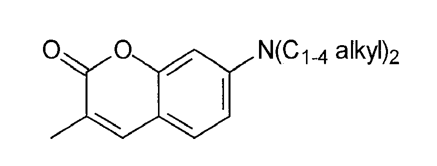

In a further embodiment the fluorophor compound has the general formula (III) or (IV): 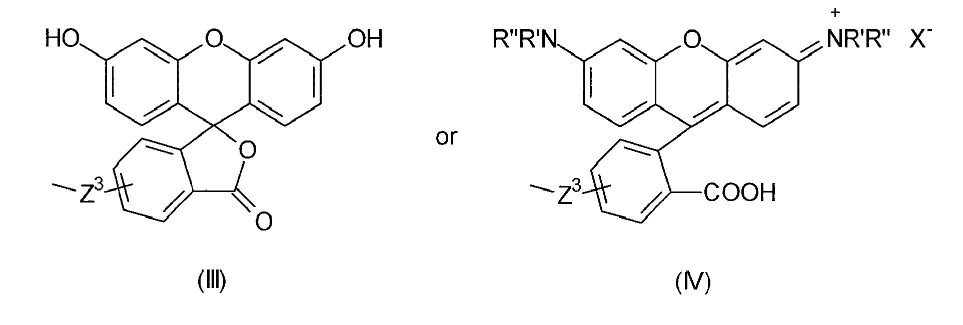 wherein
Z³ is -CH₂-, -C(O)- or -C(S)-, preferably -C(O)-.
R' and R" are independently selected for each occurrence and are selected from the group consisting of H and C₁₋₄ alkyl.

X can be any suitable anion and is preferably a halogen anion such as chloride.

The groups having the formula (III) or (IV) can be substituted in any available position by one or more substituents. The substituents are not particularly limited as long as they do not interfer with the detection of phosphorylation. Possible substituents include C₁₋₄ alkyl groups, OH, CF₃, NH₂, NH(C₁₋₄ alkyl), N(C₁₋₄ alkyl)₂, NO₂, Hal, SO₃H and phenyl. Preferred substituents are Hal, C₁₋₄ alkyl and SO₃H. Most preferably the groups having the formula (III) are unsubstituted. The groups having the formula (IV) are preferably unsubstituted or are substituted by C₁₋₄ alkyl.

Preferred cations which are suitable in the present invention include

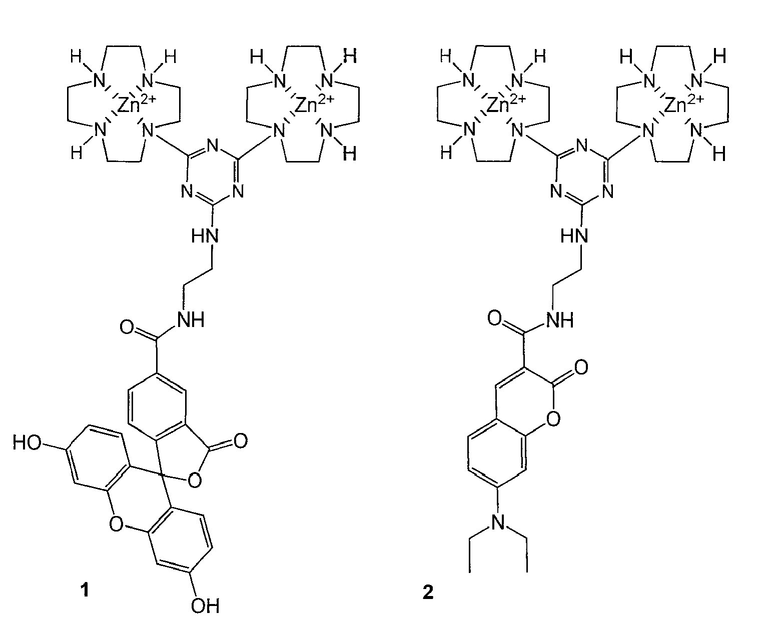

The compounds of the formula (I) can be synthesized as follows.

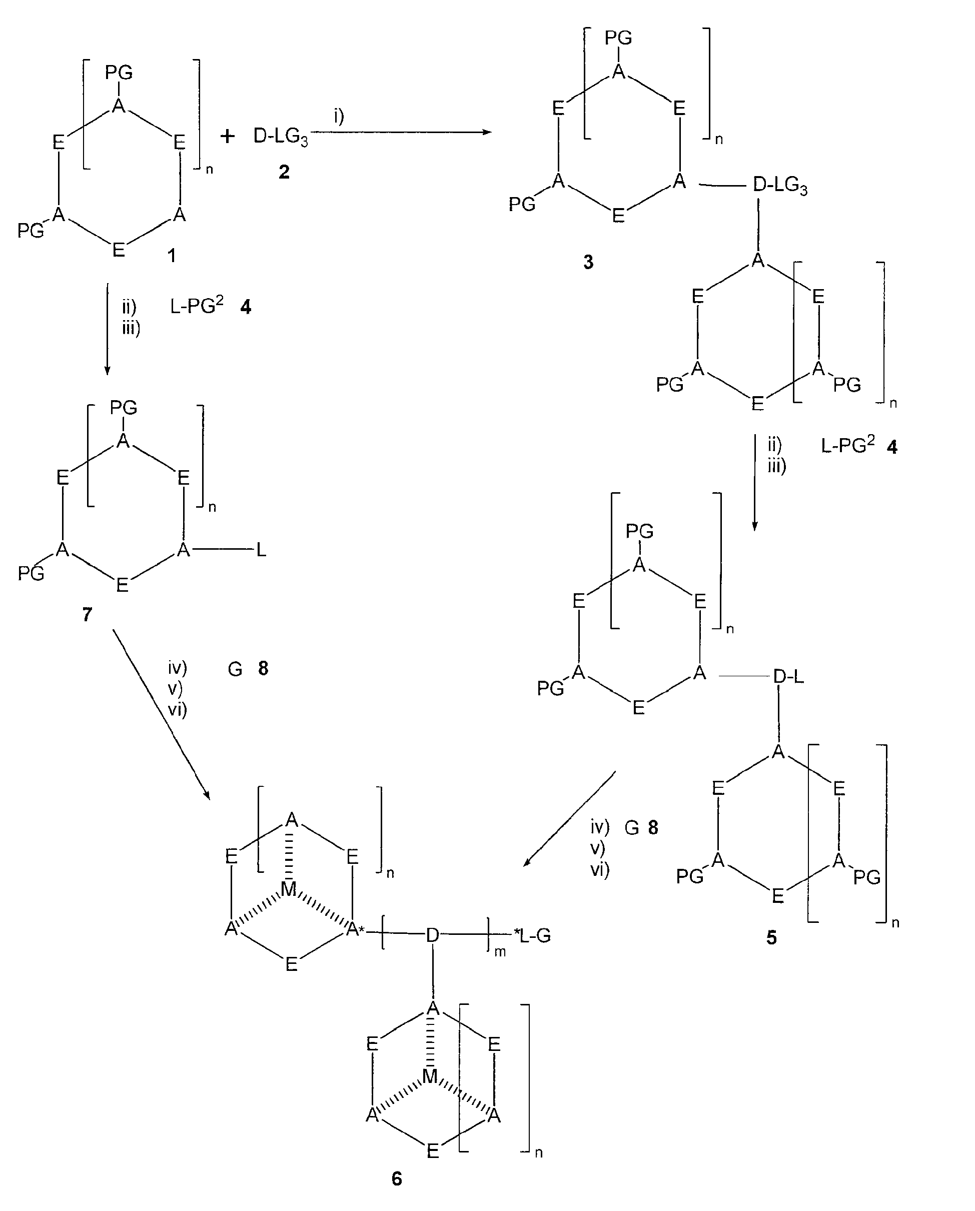

In reaction (i) compound 1 is reacted with compound 2. The reaction is usually conducted in an aprotic solvent. Examples of aprotic solvents include THF and dioxane. Preferably dioxane is chosen. The reaction is usually conducted at a temperature in the range of 60 to 120 °C, preferably from 800 to 100 °C. A base is typically employed in this reaction step. Examples of typical bases include DIEA, KOH, NaOH and K₂CO₃, preferably K₂CO₃ is used.

The reaction product is usually isolated before it is employed in reaction (ii). The isolation procedure is not particularly limited. Typically the reaction product will be separated from the solvent by removing the solvent under reduced pressure and subsequent column chromatography.

In reaction (ii) compound 3 or compound 1 is reacted with compound 4. The reaction is usually conductedj in an aprotic solvent. Examples of aprotic solvents include THF and dioxane. Preferably dioxane is chosen. The reaction is usually conducted at a temperature in the range of 100 to 140 °C, preferably from 100 to 120 °C. A base is typically employed in this reaction step. Examples of typical bases include DIEA, KOH, NaOH and K₂CO₃, preferably K₂CO₃ is used. The reaction product is usually isolated before it is employed in reaction (iii). The isolation procedure is not particularly limited. Typically the reaction product will be separated from the solvent by removing the solvent under reduced pressure and subsequent column chromatography.

In reaction (iii) the protecting group PG² of the linker is removed if D contains a primary amine, otherwise no protecting group is required. Protecting groups can be Z, Boc, and Fmoc. Preferably, the protecting group is Boc. The Boc group can be removed by addition of acid. Acids can be HCl, TFA and H₂SO₄. Preferably, the acid is TFA. The reaction is usually conducted in an aprotic solvent. Examples of aprotic solvents include DCM, Et₂O and dioxane. Preferably DCM is chosen. The reaction is usually conducted at room temperature. The reaction product is usually isolated before it is employed in reaction (iv). The isolation procedure is not particularly limited. Typically the reaction product will be separated from the solvent and the acid by removing the solvent and the acid under reduced pressure.

In reaction (iv) compound 4 or compound 7 is reacted with compound 8. Preferably, the coupling is conducted between a primary amine and a carboxylate, in L and G, respectively. The reaction can be performed using HOBt or HOAt, DIEA and HATU, PyBop, Bop, TBTU or HBTU. Typically, HOBt, TBTU and DIEA are employed. The reaction is usually conducted in an aprotic solvent. Examples of aprotic solvents include THF and dioxane. Preferably dioxane is chosen. The reaction is usually conducted at a temperature in the range of -10 to 25 °C, preferably from 0 to 10 °C. The reaction product is usually isolated before it is employed in reaction (v). The isolation procedure is not particularly limited. Typically the reaction product will be separated from the solvent by removing the solvent under reduced pressure and subsequent column chromatography.

In reaction (v) the protecting groups PG are removed if A is a secondary amine, otherwise no protecting group is required. Protecting groups can be Z, Boc, and Fmoc. Preferably, the protecting group is Boc. The Boc group can be removed by addition of acid. Acids can be HCl, TFA and H₂SO₄. Preferably, the acid is TFA. The reaction is usually conducted in an aprotic solvent. Examples of aprotic solvents include DCM, Et₂O and dioxane. Preferably DCM is chosen. The reaction is usually conducted at room temperature. The reaction product is usually isolated before it is employed in reaction (iv). The isolation procedure is not particularly limited. Typically the reaction product will be separated from the solvent and the acid by removing the solvent and the acid under reduced pressure.

In reaction (vi) the free ligand is complexed by the metal cation M. M can be introduced in the form of soluble salts, such as nitrates or perchlorates. Preferably, M is introduced as the perchlorate. The reaction is usually conducted in a protic solvent. Examples of protic solvents include water and C₁₋₄ alkanols such as EtOH and MeOH. Preferably water is chosen. The reaction is usually conducted at a temperature in the range of 60 to 100 °C, preferably from 80 to 90 °C. The reaction product is usually isolated by removing the solvent under reduced pressure.

The present invention furthermore relates to a method of detecting a phosphorylated peptide or phosphorylated polypeptide, the method comprises: (a) bringing into contact said phosphorylated peptide or phosphorylated polypeptide with a compound according to the invention; and (b) detecting a complex comprising said phosphorylated peptide or phosphorylated polypeptide and said compound, thereby detecting said phosphorylated peptide or phosphorylated polypeptide.

The term "peptide" as used herein describes a group of molecules which consist of 30 or less than 30 amino acids. The term "polypeptide" as used herein describes a group of molecules which consist of more than 30 amino acids. In accordance with the invention, the group of polypeptides comprises "proteins" as long as the proteins consist of a single polypeptide. Also in line with the definition the term "polypeptide" describes fragments of proteins as long as these fragments consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. By detecting a peptide or polypeptide which is comprised in such a multimer, the method of the invention also serves to detect such multimers where applicable. The terms "polypeptide" and "protein" also refer to naturally modified polypeptides/proteins wherein the modification is effected e.g. by glycosylation, acetylation, phosphorylation and the like. Such modifications are well known in the art.

The term "phosphorylated" in conjunction with peptides, polypeptides and proteins is known in the art. As stated above, the term refers to modified forms of peptides and polypeptides, the modified forms being **characterized in that** one or more phosphate moieties are attached, usually covalently attached, to the peptide or polypeptide. Common sites of attachment are the amino acids bearing hydroxyl groups, in particular Ser, Thr and Tyr. Phosphorylation involves the formation of a phosphate ester between the phosphate and the hydroxyl group of Ser, Thr and Tyr. In case more than one residue amenable to phosphorylation is comprised in a peptide or polypeptide, different phosphorylated forms may occur which differ in the number of phosphate moieties. For example, a single amino acid, a subset of residues amenable to phosphorylation, or all residues amenable to phosphorylation may be phosphorylated. The method of the invention is suitable to detect all these forms, wherein sensitivity of the method may increase with the number of phosphate moieties being bound to the peptide or polypeptide. Preferably, also the degree of phosphorylation, i.e., the (average) number of phosphates per peptide or polypeptide can be determined. Generally, fluorescence intensity will be proportional to the number of phosphorylated sites. Where necessary, the method of determining the degree of phosphorylation can be calibrated without further ado. Generally, binding of the compounds according to the invention will preferentially occur to surface-exposed residues. As a rule, amino acids bearing hydroxyl groups, owing to their hydrophilicity, are preferentially surface exposed on proteins, polypeptides, and peptides.

The term "detecting" according to the invention relates to determining presence or absence of the analyte. It may furthermore comprise quantitation. Quantitation may rely on the linear relation between fluorescence emission intensity and concentration, which generally applies with good approximation over a wide range of concentrations. Moreover, the skilled person can calibrate the quantitation process without further ado, for example by using dilution series of standard compounds.

The recited "bringing into contact" is performed under conditions which allow formation of a complex comprising the phosphorylated peptide and the compound of the invention, or of a complex comprising the phosphorylated polypeptide and the compound of the invention, respectively. In other words, "bringing into contact" refers to bringing about conditions under which peptide or polypeptide molecules and compounds of the invention are in such spatial proximity that complex formation may occur. Such conditions are known in the art or can be determined by the skilled person without further ado. In particular, suitable conditions include aqueous solutions of the peptide or polypeptide, for example buffered aqueous solutions, to which the compounds according to the invention are added.

Buffers are well known in the art and the skilled person is aware of appropriate buffers in dependency of the substances being assayed. Common buffers comprise (pKₐ values in brackets) H₃PO₄ / NaH₂PO₄ (pK_{a,1} = 2.12), glycine (pK_{a,1} = 2.34), acetic acid (4.75), citric acid (4.76), MES (6.15), cacodylic acid (6.27), H₂CO₃ / NaHCO₃ (pK_{a,1} = 6.37), bis-Tris (6.50), ADA (6.60), bis-Tris propane (pK_{a,1} = 6.80), PIPES (6.80), ACES (6.90), imidazole (7.00), BES (7.15), MOPS (7.20), NaH₂PO₄ / Na₂HPO₄ (pK_{a,2} = 7.21), TES (7.50), HEPES (7.55), HEPPSO (7.80), triethanolamine (7.80), tricine (8.10), Tris (8.10), glycine amide (8.20), bicine (8.35), glycylglycine (pK_{a,2} = 8.40), TAPS (8.40), bis-Tris propane (pK_{a,2} = 9.00), boric acid (H₃BO₃ / Na₂B₄O₇) (9.24), CHES (9.50), glycine (pK_{a,2} = 9.60), NaHCO₃ / Na₂CO₃ (pK_{a,2} = 10.25), CAPS (10.40) and Na₂HPO₄ / Na₃PO₄ (pK_{a,3} = 12.67). Furthermore, ionic strength may be adjusted, e.g., by the addition of sodium chloride and/or potassium chloride. Preferred concentrations of sodium chloride are from 0 to 2 M, preferably from 100 to 200 mM. Examples of buffers comprising sodium chloride include PBS (phosphate buffered saline) containing 1.37 M NaCl, 27 mM KCI, 43 mM Na₂HPO₄·7H₂O and 14 mM KH₂PO₄ in the 10-fold aqueous stock solution, which is adjusted to pH 7.3; SSC containing 3 M NaCl and 0.3 M sodium citrate in 20-fold aqueous stock solution, which is adjusted to pH 7.0; and STE (saline Tris EDTA) containing 10 mM Tris base, 10 mM NaCl and 1mM EDTA (acid). Alternatively, sodium chloride is absent from the buffer preparation. Examples for common buffer preparations without sodium or potassium chloride are TAE (Tris acetate EDTA) containing 2 M Tris acetate and 0.1 M EDTA in the 50-fold aqueous stock solution at pH 8.5; TBE (Tris borate EDTA) containing 0.89 M Tris base, 0.89 M boric acid and 0.02 M EDTA in the 10-fold aqueous stock solution at pH 8.0; and TE (Tris EDTA) containing 10 mM Tris base and 1 mM EDTA (acid) at pH 7.5.

Also, and as described in the examples, the compounds of the invention may be dissolved in de-ionized water. To the extent the peptide or polypeptide is soluble in de-ionized water, suitable conditions according to the invention also include solutions of the peptide or polypeptide and the compound of the invention in de-ionized water.

Typically, the polypeptide or peptide is comprised in a sample. The sample may be taken from an organism, a cell in culture, from the environment, or from a patient or subject suspected to suffer from a disease. The sample may be a crude cellular extract or may be purified to different degrees (for details see below). The purified sample may be an aqueous solution or aqueous buffered solution of the polypeptide or peptide.

The polypeptide or peptide may also be part of a cell, tissue or organism. The cell may be a cell in culture. The tissue may be in vitro, ex vivo or part of an organism. The organism may be a plant or an animal, animals including humans. When applied to cells, tissue or organisms, the methods according to the invention may also provide information as regards the spatial distribution of phosphorylated polypeptides or peptides. When performed repeatedly at different points in time to the same cell, tissue or organism, the methods according to the invention may also provide information as regards the time-dependency of phosphorylation. Moreover, spatio-temporal patterns of phosphorylation may be determined.

The term "complex" is known in the art and refers to non-covalent association of two or more molecules. In the context of the method according to the invention, the term "complex" refers to a complex comprising or consisting of a peptide molecule or polypeptide molecule and one or more compounds according to the invention, i.e., one or more probe molecules. As stated above, the peptide or polypeptide may be comprised in a multimer of polypeptides. The number of probe molecules comprised in the complex will depend on the amount of the compound being used and the number of phosphorylated sites which are accessible. In case more than one probe molecule associates with a peptide or polypeptide, these probe molecules may be of the same or different molecular species.

The detecting of the complex may be performed by any means and methods suitable to detect formation of a complex. Such methods include, for example, the determination of the change of amount or concentration of the free (uncomplexed) form of peptide, polypeptide and/or compound according to the invention.

Preferred methods of detecting the complex make use of the fluorescent properties of the compounds of the invention. For example, if detection is performed on or in a support such as a gel (for details see further below), the peptides or polypeptides may not be distributed homogeneously within the gel, for example after performing electrophoresis. After staining of the gel with compounds of the invention, washing may be performed in order to remove unbound compounds. In such a case, detection involves detecting the fluorescent region or band on the gel. The fluorescent region or band corresponds to a phosphorylated peptide or polypeptide.

Detection may be performed with the nake or eye and/or with the help of photographic devices, optionally in combination with microscopic devices. Analysis of the obtaining images may be performed with the aid of computers having suitable software implemented thereon. Computer-based image analysis is well known in the art and commonly referred to as image analysis.

In a preferred embodiment, the detecting of said complex comprises determining whether, upon said bringing into contact, (i) fluorescence emission of said compound increases, and/or (ii) the wavelength of the fluorescence emission maximum of said compound is shifted, wherein an increase of fluorescence emission and/or shift of the wavelength of the fluorescence emission maximum is indicative of the presence of said complex. The effect according to (i) is an overall change in fluorescence intensity. The effect according to (ii) is change of the fluorescence emission spectrum.

The present invention furthermore provides a method of detecting and differentiating phosphorylated and unphosphorylated peptides or polypeptides, the method comprising: (a) bringing into contact said peptides or polypeptides with a compound; and (b) determining (i) whether fluorescence emission being characteristic of a complex of said compound with phosphorylated peptides or phosphorylated polypeptides occurs; and (ii) whether fluorescence emission being characteristic of a complex of said compound with unphosphorylated peptides or unphosphorylated polypeptides occurs, thereby detecting and differentiating phosphorylated and unphosphorylated peptides or polypeptides.

The term "differentiating" refers to distinguishing between phosphorylated and unphosphorylated peptides and polypeptides. Phosphorylated and unphosphorylated polypeptides (or peptides) may be polypeptides (or peptides) with different amino acid sequences or may be polypeptides (or peptides) with the same amino acid sequence. In the latter case, phosphorylated and unphosphorylated forms of an otherwise identical, at least with regard to the amino acid sequence, polypeptide (or peptide) are differentiated.

The term "fluorescence emission being characteristic of a complex [...]" refers to a reference state for comparison. This reference state may be the fluorescence emission of a particular peptide or polypeptide in phosphorylated and unphosphorylated form, respectively. This may be particularly useful for those embodiments where differentiating between phosphorylated and unphosphorylated forms of this particular peptide or polypeptide is desired. However, the reference state is not so limited. The reference states may be mixtures of polypeptides (or peptides), or polypeptides (or peptides), wherein phosphorylated and unphosphorylated reference states have different amino acid sequences.

This embodiment exploits the following properties of the compounds of the invention. The compounds of the invention are capable of forming a complex also with binding sites on a peptide or polypeptide which are not phosphorylated sites. The complexes of compounds of the invention with non-phosphate binding sites differ from complexes with phosphorylated sites with regard to fluorescence emission.

In preferred embodiments, and depending on the choice of the fluorophore, the overall fluorescence emission of the unphosphorylated peptide or polypeptide is different in intensity, preferably lower in intensity as compared to the phosphorylated polypeptide or peptide, and/or the fluorescence emission, while being of similar magnitude, exhibits an emission maximum at a different wavelength. The latter phenomenon, i.e., the dependency of the fluorescence emission spectrum on the environment, is also referred to as solvatochromic effect. Fluorophores according to formula (II) exhibit a solvatochromic effect, whereas fluorophores according to formula (III) exhibit lower fluorescence intensity when bound to a binding site which is not a phosphorylated site. Without wishing to be bound by a specific theory, the latter effect is attributed to quenching which occurs at non-phosphate binding sites.

Accordingly, in a preferred embodiment of the method of detecting and differentiating phosphorylated and unphosphorylated peptides or polypeptides, fluorescence emission according to (i) differs from fluorescence emission according to (ii) with regard to the wavelength of the fluorescence emission maximum. In a particularly preferred embodiment, the fluorophor comprised in said compound is of the general formula (II).

In preferred embodiments of the methods of the invention, the methods are effected on or in a support.

Supports include any type of solid material including glass, polymers or metals or combinations thereof. Supports may have the form of flat sheets such as membranes, or may be three-dimensional objects with a certain thickness such as gels. A support may serve to immobilize peptides or polypeptides, for example on one or more sites or areas within the support. In the latter case, polypeptides or peptides are not distributed homogeneously within the support. Preferably, said peptide or polypeptide is capable of binding or adhering to said support. Binding or adhering of peptides or polypeptides to the support material generally occurs non-covalently. Peptides and polypeptides may be bound at the surface of the support (e.g., of a membrane) or within the support, e.g., within a gel. By immobilizing the polypeptide or peptide, a support offers distinct advantages over detection in solution. Polypeptides or peptides are confined to their respective sites of immobilization and cannot diffuse freely. If immobilization occurs at specific sites or areas, the locations of polypeptides or peptides may correspond to or reveal information on further properties of the polypeptides or peptides, including their identity. For example, in those embodiments where the support is an array, polypeptides or peptides are deposited at defined sites or areas within the support. Knowledge of the site where a polypeptide or peptide has been deposited provides knowledge of its identity. Detection of fluorescence at those sites by methods of the invention allows information on the phosphorylation states of the polypeptide or peptide immobilized at that particular site to be obtained. Alternatively, polypeptides or peptides may be confined to particular areas of a support as a consequence of a separation process such as electrophoresis, see below. In that case, the location of the polypeptide or peptide generally is determined by and corresponds to its molecular weight. Also, and in particular the support is a membrane or filter, the polypeptide or peptide may be confined to a particular area as a consequence of a process of transfer from another support, wherein the polypeptide or peptide is confined to a particular area within said another support. Such process of transfer is known in the art and includes blotting. When blotting precedes performing the method of the invention, this embodiment of the method is also referred to as "on-blot".

Supports furthermore include cells, tissues or non-human organisms, wherein said cells, tissues or non-human organisms are attached to or bound to a support. Cells, tissues or organisms may be fixated. Fixation is a means of preparing cells, tissues or organisms and is known in the art. Preferred embodiments of cells, tissues and organisms are described herein above. Tissues may be provided as cross-sections.

When performing the method of the invention on or in a support, the step of bringing into contact may also be referred to as "staining". Preferred or exemplified conditions for staining, in particular for staining of gel, can be found in the enclosed examples.

Preferred supports are selected from (a) gels; (b) membranes; (c) filters; and (d) arrays. Gels, membranes and filters are commonly used in the field of molecular biology and analytics.

A gel is a solid, jelly-like material that can have properties ranging from soft and weak to hard and tough. Gels are defined as a substantially dilute crosslinked system, which exhibits no flow when in the steady-state. By weight, gels are mostly liquid, yet they behave like solids due to a three-dimensional crosslinked network within the liquid. It is the crosslinks within the fluid that give a gel its structure (hardness) and contribute to stickiness (tack).

Preferably, the gel is a polyacrylamide gel. A polyacrylamide gel is a separation matrix used in electrophoresis of biomolecules, such as proteins or nucleic acids. A polyacrylamide gel may comprise a stacking gel and a resolving gel. Typically resolving gels are made in 6%, 8%, 10%, 12% or 15%. Stacking gel (5%) is poured on top of the resolving gel and a gel comb (which forms the wells and defines the lanes where proteins, sample buffer and ladders will be placed) is inserted. The percentage chosen depends on the size of the protein that one wishes to identify or probe in the sample. The smaller the known molecular weight, the higher the percentage that should be used.

Alternatively, the gel may for example be an agarose gel.

Gels such as polyacrylamide gels may comprise a denaturant. Denaturants include sodium dodecyl sulphate (SDS), urea and guanidinium chloride. Furthermore, agents which reduce the disulfide bonds present in many polypeptides may be used as denaturants. Agents reducing disulfide bonds include 2-mercaptoethanol and dithiothreitol.

Denaturants may be used to unfold polypeptides or reduce the amount of tertiary structure. As a consequence, the migration behaviour of the polypeptide in the gel is predominantly, or at least to greater extent than in the folded state, governed by the molecular weight.

Membranes and filters are usually provided as flat sheets made of various materials, preferably of material to which polypeptides or peptides can adhere or bind.

Preferred membranes are polyvinylidene difluoride (PVDF) membranes and nitrocellulose membranes. These membranes are known in the art, commonly used for Western blotting, and are available from various manufacturers.

In preferred embodiments of methods using a gel, electrophoresis of said peptide or polypeptide is performed prior to, concomitantly with, or after step (a). Gel electrophoresis is well known in the art and is a technique used for the separation of biomolecules, including peptides and polypeptides, using an electric current applied to a gel matrix. Gel electrophoresis permits the separation of peptides and polypeptides according to their size. In certain instances, also different degrees of phosphorylation of a peptide or polypeptide may give rise to discernible differences in migration during gel electrophoresis. This embodiment of the invention is also referred to as "in gel" method.

The term "array" is well known in the art. In the context of the present invention it refers to a support having different species of polypeptides or peptides immobilized thereto. Preferably, each polypeptide or peptide has its own distinct site of immobilization, thereby facilitating assignment of position on the array to identity of the polypeptides or peptides. Supports suitable for the immbilization of polypeptides or peptides are known in the art (see e.g., Stoll, D., Bachmann, J., Templin, M.F., Joos, T.O. (2004) Microarray technology: an increasing variety of screening tools for proteomic research Targets 3: 24-31; Cahill, D.J., Nordhoff, E. (2003) Protein arrays and their role in proteomics. Adv Biochem Eng Biotechnol. 83:177-87.; Gershon, D. (2003) Proteomics technologies: probing the proteome Nature 424:581-7.

In further embodiments of the methods of the invention, the methods are effected in solution. Performing the methods in solution is an alternative to performing them using a support.

The present invention furthermore provides the use of a compound of the present invention for detecting a phosphorylated peptide or phosphorylated polypeptide.

Also provided is the use of a compound of the present invention for detecting and differentiating phosphorylated and unphosphorylated forms of a peptide or polypeptide.

The invention furthermore relates to a kit comprising one or more compounds of the present invention.

Preferably the kit further comprises a manual, the manual comprising instructions for performing at least one of the methods according to the invention.

The present invention furthermore relates to a diagnostic composition comprising one or more compounds of the present invention. The diagnostic composition may furthermore comprise a carrier, diluent or excipient. For example, the diagnostic composition may be an aqueous solution or buffered aqueous solution of one or more compounds of the invention. Buffers are well known in the art and further detailed herein above.

Also provided is a method of diagnosing a disease characterized by presence of an aberrant amount of a phosphorylated peptide or phosphorylated polypeptide, the method comprising (a) bringing into contact a sample obtained from a subject suspected to suffer from said disease with a compound of the present invention; and (b) determining whether the fluorescence emission of said sample differs from the fluorescence emission of a control sample.

A "control sample" may be a sample which has been taken from a healthy individual or a sample which reflects the phosphorylation status of the polypeptide in a healthy individual. The term "aberrant amount" refers to both an aberrant absolute amount of the peptide or polypeptide in question, wherein the ratio of phosphorylated and unphosphorylated form do not deviate from the ratio found in healthy individuals, as well as to aberrant ratios (relative amounts). The latter phenomenon includes hyper- and hypophosphorylation.

Preferably, the sample is purified prior to performing step (a). Purification preferably includes removal of insoluble material formed upon lysis of the cells of the sample. Purification may furthermore comprise removal of nucleic acids and/or enrichment of polypeptides or certain groups of polypeptides. Also envisaged is purification which delivers the polypeptide in question, be it phosphorylated or not, phosphorylated to different degrees, or mixtures thereof, in substantially pure form. Suitable means and methods for purification are well known to the person skilled in art and include chromatography such as size exclusion chromatography, ion exchange chromatography and affinity chromatography.

Cellular signaling involves numerous phophorylation and de-phosphorylation events. Enzymes capable of adding phosphate residues to polypeptides and peptides include kinases and form a prominent class of drug targets. Aberrant growth factor signaling, for example, may give rise to aberrantly high kinase activity, which in turn may entail aberrant amounts of phosphorylated peptides or phosphorylated polypeptides as defined above. Since aberrant growth factor signaling relates to hyperproliferation and neoplasms, cancer is one of the diseases which may be diagnosed on the basis of such aberrant amounts.

The present invention will now be illustrated by the following examples. However, the present invention is not to be construed as being limited thereto.

### EXAMPLES

### Example 1: Synthesis of probes 1 and 2

All reactions were performed under an inert atmosphere of N₂ using standard Schlenk techniques if not otherwise stated.

A Varian Cary BIO 50 UV/VIS/NIR Spectrometer was used. A 1 cm quartz cell was purchased from Hellma and Uvasol solvents were obtained from Merck or Baker. Absorption and emission maxima are given in nm, molar absorptivities (ε) are given in M⁻¹ cm⁻¹.

IR spectra were recorded on a Bio-Rad FT-IR FTS 155 and a Bio-Rad FTS 2000 MX FT-IR using a Specac Golden Gate Mk II ATR accessory where stated.

NMR spectrometers used were: Bruker Avance 600 (¹H: 600.1 MHz, ¹³C: 150.1 MHz, T = 300 K), Bruker Avance 400 (¹H: 400.1 MHz, ¹³C: 100.6 MHz, T = 300 K) and Bruker Avance 300 (¹H: 300.1 MHz, ¹³C: 75.5 MHz, T = 300 K). The chemical shifts are reported in δ [ppm] relative to external standards (solvent residual peak). The spectra were analyzed by first order, the coupling constants are given in Hertz [Hz]. Characterization of the signals: s = singlet, d = doublet, t = triplet, q = quartet, m = multiplet, bs = broad singlet, psq = pseudo quintet, dd = double doublet, dt = double triplet, ddd = double double doublet. Integration is determined as the relative number of atoms. Assignment of signals in ¹³C-spectra was determined with DEPT-technique (pulse angle: 135 °) and given as (+) for CH₃ or CH, (-) for CH₂ and (C_{quat}) for quaternary C. Error of reported values: chemical shift: 0.01 ppm for ¹H-NMR, 0.1 ppm for ¹³C-NMR and 0.1 Hz for coupling constants. The solvent used is reported for each spectrum.

Mass spectra were recorded on Varian CH-5 (EI), Finnigan MAT 95 (Cl; FAB and FD) and Finnigan MAT TSQ 7000 (ESI). Xenon served as the ionization gas for FAB.

Melting Points were determined on a Büchi SMP-20 melting point apparatus and are uncorrected. TLC analyses were performed on silica gel 60 F-254 with a 0.2 mm layer thickness. Detection was via UV light at 254 nm / 366 nm or by staining with ninhydrin in EtOH.

For preparative column-chromatography, Merck Geduran SI 60 silica gel was used.

Commercially available solvents of standard quality were used. Unless otherwise stated, purification and drying was done according to accepted general procedures (15, 16). Compounds **3** (17) and **5** (18) were synthesized according to literature known procedures.

### Synthesis of probes 1 and 2

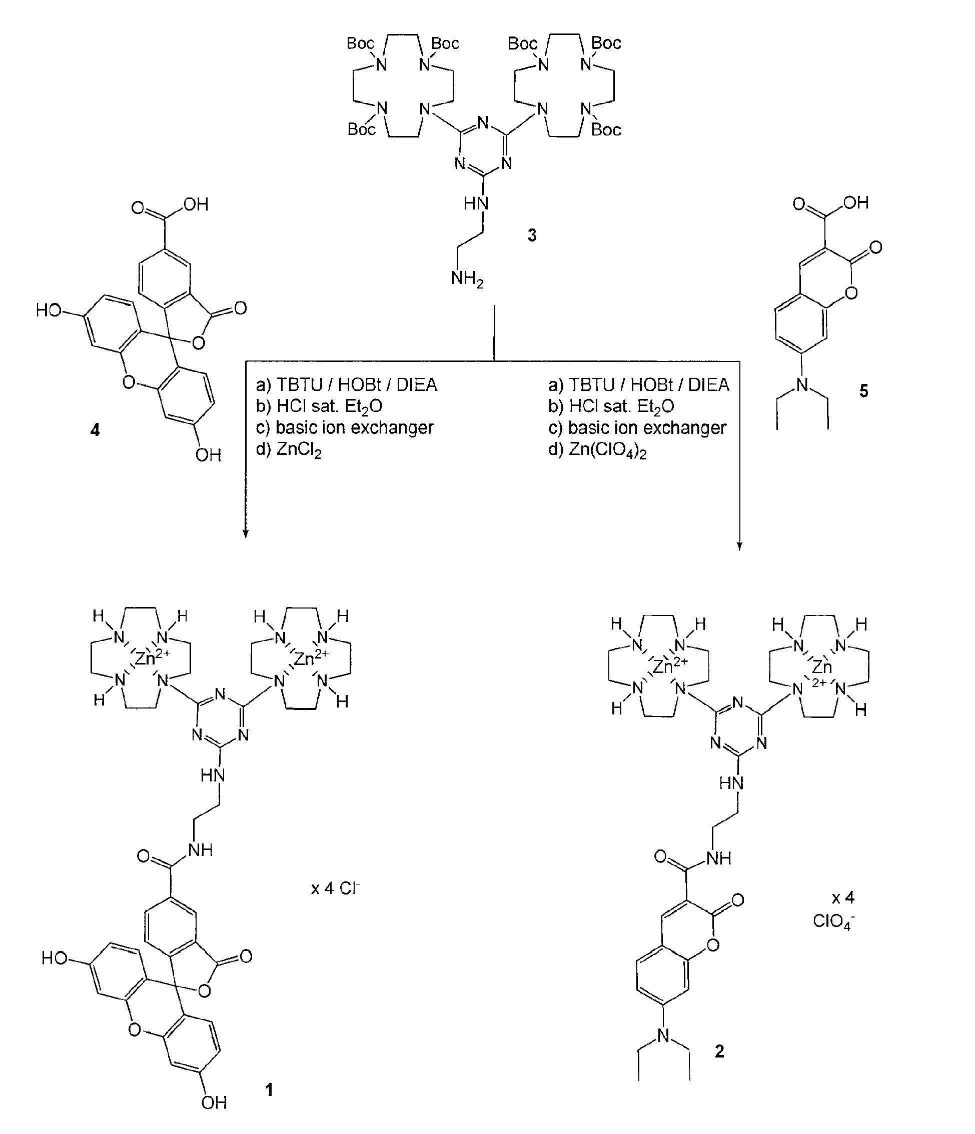

*Regioisomeric 1,4,7-tri-tert-butyl 10,10'-{6-[2-(3',6'-dihydroxy-3-oxo-3h-spiro[isobenzofuran-1,9'-xanthene]-5*/*6-ylcarboxamido)ethylamino]1,3,5-triazine-2,4-diyl}bis(1,4,7,10-tetraazacyclododecane-1,4,7-tricarboxylate* (**6**):

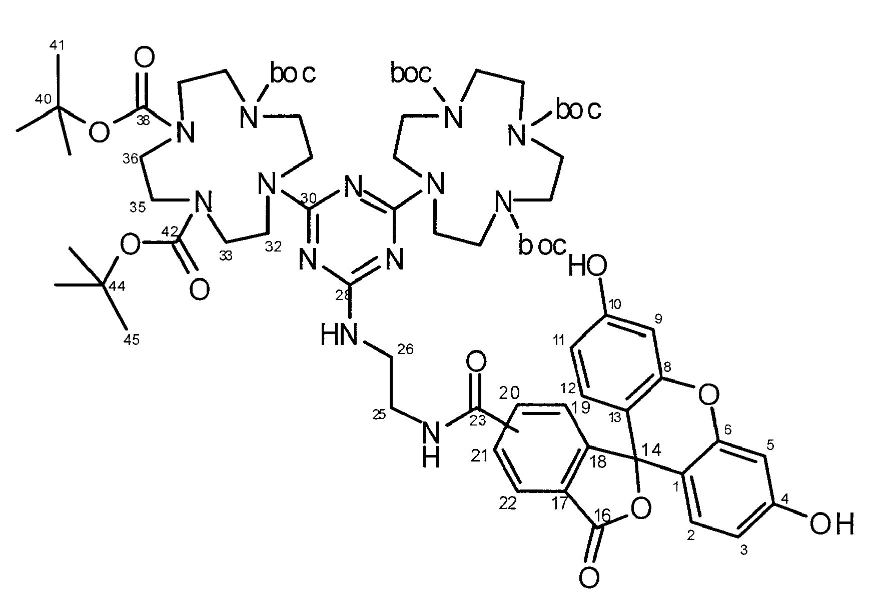

In a nitrogen flushed round bottom flask, regioisomeric 5-,6-carboxyfluorescein (0.15 g, 0.40 mmol) was dissolved in 10 mL of a mixture of dichloromethane and DMF (2:1), then DIPEA (0.17 g, 180 µL, 1.32 mmol) and HOBt monohydrate (0.07 g, 0.48 mmol) were added. While stirring, the mixture was cooled to 0 °C in an ice water bath to add TBTU (0.15 g, 0.48 mmol). After 30 min, 1,4,7-tri-tert-butyl 10,10'-(6-(2-aminoethylamino)-1,3,5-triazine-2,4-diyl) bis (1,4,7,10-tetraazacyclo-dodecane-1,4,7-tricarboxylate) (**3**) (0.47 g, 0.44 mmol) was added in portions to the flask and the reaction mixture was heated to 40 °C for 2 h. Subsequently, the solvents were evaporated and the crude product was purified by column chromatography (CHCl₃ : MeOH = 15 : 1) on flash-silica-gel yielding compound **6** as an orange amorphous solid (0.34 g, 0.23 mmol, 59%).

**MP:** 207°C
**¹H-NMR** (600 MHz, MeOD, COSY, ROESY, HSQC, HMBC): δ [ppm] = 8.36 (s, 0.7H, 22a), 8.13 (d, *³J_{H,H}* = 7.91 Hz, 0.7H, 20a), 8.07 (d, *³J_{H,H}* = 8.08 Hz, 0.3H, 21 b), 8.03 (d, *³J*_{*H,*H} = 8.04 Hz, 0.3H, 22b), 7.61 (s, 0.3H, 19b), 7.24 (d, *³J_{H,H}* = 8.02 Hz, 0.7H, 19a), 6.68 (dd, *³J_{H,H}* = 1.75 Hz, *⁵J_{H,H}* = 1.75 Hz, 2H, 2+12), 6.57 (dd, *⁴J_{H,H}* = 8.71 Hz, *⁵J_{H,H}* = 8.71 Hz, 2H, 5+9), 6.52 (dd, *³J_{H,H}* = 8.72 Hz, *⁴J_{N,H}* = 2.31 Hz, 2H, 3+19), 3.99-3.20 (m, 36H, 25a/b, 26a/b, 32+33+35+36), 1.45-1.37 (m, 54H, 41+45)
**¹³C-NMR** (150 MHz, MeOD, COSY, ROESY, HSQC, HMBC): δ [ppm] = 170.4 (C_{quat.}, 0.3C, 16b), 170.3 (C_{quat.}, 0.7C, 16a), 168.5 (C_{quat.}, 0.7C, 23a), 168.3 (C_{quat.}, 0.3C, 23b), 167.8 (C_{quat.}, 2C, 30), 167.5 (Cq_{uat.}, 0.7C, 28a), 167.3 (C_{quat.}, 0.3C, 28b), 161.3 (C_{quat.}, 2C, 4+10), 158.0 (C_{quat.}, 6C, 38+42), 156.7 (C_{quat.}, 0.7C, 18a), 154.4 (C_{quat.}, 0.3C, 18b), 153.9 (C_{quat.}, 2C, 6+8), 142.4 (C_{quat.}, 0.3C, 20b), 138.0 (C_{quat.}, 0.7C, 21a), 135.5 (+, 0.7C, 20a), 130.2 (+, 0.3C, 21b), 130.1 (+, 2C, 5+9), 128.5 (C_{quat.}, 1C, 17a/b), 126.1 (+, 0.3C, 22b), 125.6 (+, 0.7C, 19a), 124.8 (+, 0.7C, 22a), 124.0 (+, 0.3C, 19b), 113.7 (+, 2C, 3+11), 110.8 (C_{quat.}, 2C, 1+13), 103.7 (+, 2C, 2+12), 81.5 (C_{quat.}, 2C, 38), 81.4 (C_{quat.}, 4C, 42), 79.4 (C_{quat.}, 1C, 14), 51.4 (-, 16C, 32+33+35+36), 41.7 (-, 0.7C, 25a), 41.5 (-, 0.3C, 25b), 40.9 (-, 0.7C, 26a), 40.8 (-, 0.3C, 26b), 29.9 (+, 6C, 41), 28.8 (+, 12C, 45)
**IR** (ATR) [cm⁻¹]: ν = 3318, 2971, 2935, 1766, 1689, 1665, 1539, 1466, 1410, 1365, 1247, 1157,1024,852,811,751,665
**ES-MS** (H₂O/MeOH + 10 mmol/L NH₄Ac): m/z (%) = 720.0 (100) [M + 2H⁺], 1439.0 (5) [MH⁺]
**UV** (MeCN): λ (ε) = 454 (2175), 481 (1876)
*Regioisomeric N-{2-[4,6-di(1,4,7,10-tetraazacyclododecan-1-yl)-1,3,5-triazin-2-ylamino]-ethyl}-3',6'-dihydroxy-3-oxo-3H-spiro[isobenzofuran-1,9'-xanthene]-5-carboxamide octachloride* (**7**):

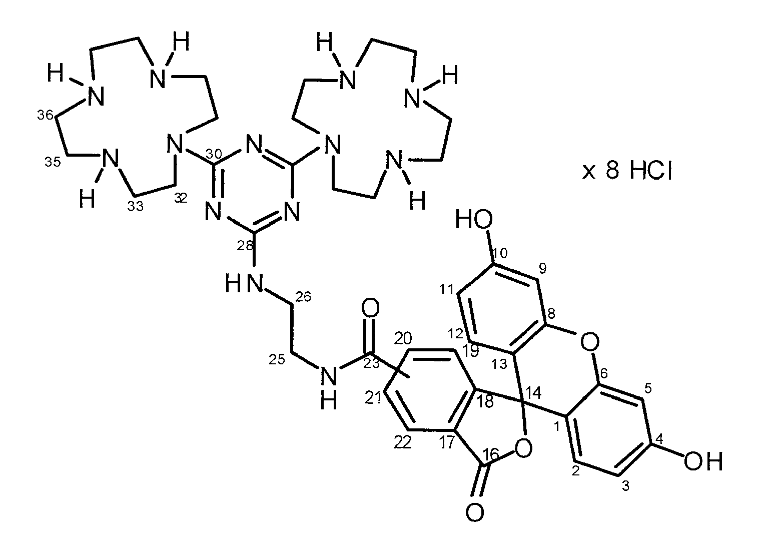

Compound **6** (80 mg, 0.05 mmol) was dissolved in as little dichloromethane as possible and cooled to 0 °C before hydrochloric acid saturated diethyl ether (1.06 mL) was added slowly to this solution. Stirring was continued for 16 h while the reaction mixture was allowed to reach room temperature. Formation of a yellowish precipitate was observed. The desired product was obtained by evaporation of the solvent in vacuum as a red amorphous solid (50 mg, 0.05 mmol, quant.), which was deprotonated by ion exchange column on weakly basic anion exchanger resin.

**¹H-NMR** (600 MHz, D₂O, COSY, HSQC, HMBC): δ [ppm] = 8.43 (s, 2H, 24), 7.97 (s, 0.6H, 22a), 7.93-7.86 (d, *³J_{H,H}* = 7.37 Hz, 0.4H, 21b), 7.85-7.79 (d, *³J_{H,H}* = 7.40 Hz, 0.4H, 22b), 7.74 (bs, 0.6H, 20a), 7.44 (s, 0.4H, 19b), 7.02 (bs, 0.6H, 19a), 6.99-6.70 (m, 2H, 2+12), 6.61-6.41 (m, 2H, 3+11), 6.41-6.13 (m, 2H, 5+9), 3.87-3.39 (m, 12H, 25+26+36), 3.27-2.88 (m, 24H, 32+33+35)
**¹³C-NMR** (150 MHz, D₂O, COSY, HSQC, HMBC): δ [ppm] = 179.9 (C_{quat.}, 2C, 30), 174.2 (C_{quat.}, 0.4C, 16b), 173.2 (C_{quat.}, 0.6C, 16a), 171.5 (C_{quat.}, 1C, 28), 170.1 (C_{quat.}, 0.4C, 23b), 169.5 (C_{quat.}, 0.6C, 23a), 167.2-166.3 (C_{quat.}, 2C, 4+10), 158.6-158.4 (C_{quat.}, 2C, 6+8), 157.9 (C_{quat.}, 0.6C, 18a), 157.2 (C_{quat.}, 0.4C, 18b), 143.7 (C_{quat.}, 0.4C, 20b), 140.7 (C_{quat.}, 0.6C, 21a), 135.6 (C_{quat.}, 0.4C, 17b), 135.2 (C_{quat.}, 0.6C, 17a), 131.9-131.5 (+, 2C, 2+12), 130.7 (+, 0.6C, 19a), 129.5 (+, 0.4C, 22b), 129.2 (+, 0.4C, 21b), 128.7 (+, 0.4C, 19b), 128.4 (+, 1.2C, 20a+22a), 123.3 (+, 2C, 3+11), 104.4 (+, 2C, 5+9), 46.5-45.2 (-, 12C, 33+35+36), 43.9 (-, 4C, 32), 40.8-40.6 (-, 1C, 25a/b), 39.9 (-, 1C, 26)
**ES-MS** (MeCN/TFA): *m*/*z* (%) = 280.1 (100) [M + 3H⁺], 419.8 (63) [M + 2H⁺], 838.5 (4) [MH⁺], 874.4 (3) [MH⁺ + HCl]

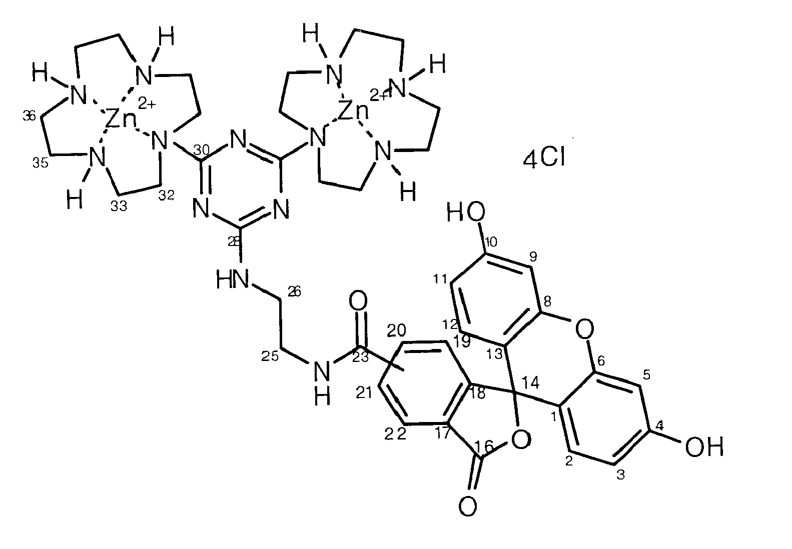

*Probe* **1**:
Deprotonated compound **7** (48 mg, 0.06 mmol) was dissolved in 2 mL of warm water, then zinc(II)-chloride (16 mg, 0.12 mmol) was added. After pH-adjustment to 7-8 with saturated NaHCO_{3, aqu.}, the mixture was heated to 100 °C for 2 h while stirring. The desired product was isolated by lyophilisation of the reaction mixture as a dark orange, amorphous solid (48 mg, 0.04 mmol, 75%).
**¹H-NMR** (600 MHz, D₂O, COSY, HSQC, HMBC): δ [ppm] = 8.14-6.06 (m, 9H, *aryl*), 4.25-2.62 (m, 35H, *amide-NH+CH₂*)
**¹³C-NMR** (150 MHz, D₂O, COSY, HSQC, HMBC): δ [ppm] = 174.1-103.2 (C_{quat.}&-, 24C, 1-23+28+30), 46.3-38.7 (-, 18C, 25+26+32-36)
**ES-MS** (H₂O/MeOH + 10 mmol/L NH₄Ac): *m*/*z* (%) = 511.4 (70) [M⁴⁺ - H⁺ + CH₃COO⁻], 481.8 (37) [M⁴⁺ - 2H⁺]
**UV** (40 mM TRIS-buffer, 10 mM MgCl₂, 2 mM DTE, pH 7.6): λ (ε) = 321 (6430), 497 (56320)
**Fluorescence** (40 mM TRIS-buffer, 10 mM MgCl₂, 2 mM DTE, pH 7.6): exc. 494 nm, emission max. 526 nm
*7-Diethylamino-2-oxo-2H-chromene-3-carboxylic acid {2-[4,6-bis-(1,4,7,10-tetraazacyclododec-1-yl)-[1,3,5]triazin-2-yl]-ethyl}-1,4,7-tricarboxylic acid tri-tert-butyl ester* (**8**): 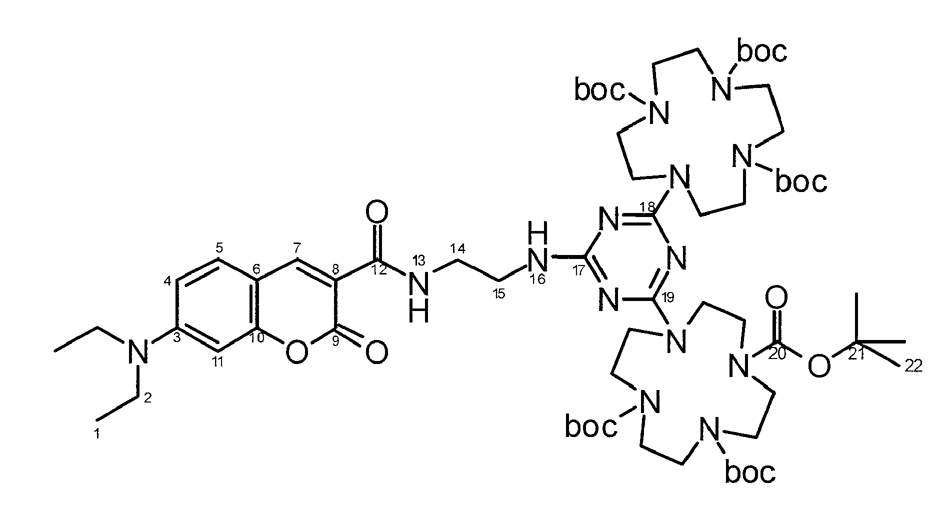 7-Diethylamino-2-oxo-2H-chromene-3-carboxylic acid (150 mg, 0.56 mmol), DIPEA (386 µL, 2.24 mmol), TBTU (203 mg, 0.63 mmol), and HOBt (97 mg, 0.63 mmol) were dissolved under nitrogen atmosphere in dry DMF (4 mL) under ice cooling and stirred for 1 h. Subsequently amine 3 (635 mg, 0.59 mmol) dissolved in DMF (2 mL) was added drop wise.

The reaction was allowed to warm to room temperature and was stirred 30 min at room temperature and 3 h at 40 °C. The reaction progress was monitored by TLC (chloroform/MeOH 97.5/2.5). After completion of the reaction the solvent was removed and the crude product was purified by flash column chromatography on flash silica gel (chloroform/MeOH 97.5/2.5; R_{f} (EE) = 0.60) yielding compound **8** (684 mg, 0.52 mmol, 93 %) as a yellow solid.

**MP**: 147 °C
**¹H-NMR** (400 MHz; CDCl₃): δ (ppm) = 1.21 (t, ³J = 7.1 Hz, 6 H, HSQC, COSY: C¹H₃), 1.40 (s, 18 H, HSQC, COSY: C²²H₃), 1.42 (s, 36 H, HSQC, COSY: C²²H₃), 3.11-3.76 (m, 36 H, C¹⁴H₂, C¹⁵H₂, cyclen-CH₂), 3.42 (q, ³J = 7.1 Hz, 4 H, HSQC, HMBC, COSY: C²H₂), 5.02 (bs, 1 H, NH¹⁶), 6.46 (d, ⁴J = 2.3 Hz, 1 H, HSQC, HMBC, COSY: C⁵H), 6.82 (dd, ³J = 8.9 Hz, ⁴J = 2.4 Hz, 1 H, HSQC, HMBC, COSY: C⁴H), 7.39 (d, ³J = 8.9 Hz, 1 H, HSQC, HMBC, COSY: C¹¹H), 8.65 (s, 1 H, HSQC, HMBC, COSY: C⁷H), 8.90 (m, 1 H, HSQC, COSY: NH¹³).
**¹³C-NMR** (100 MHz; CDCl₃): δ (ppm) = 12.4 (+, 2 C, HSQC, HMBC: C¹H₃), 28.41, 28.46 (+, 18 C, HSQC, HMBC: C²²H₃), 39.6 (-, 1 C, HSQC, HMBC: C¹⁴H₂), 40.8 (-, 1 C, HSQC, HMBC: C¹⁵H₂), 45.0 (-, 2 C, HSQC, HMBC, COSY: C²H₂), 50.2 (-, 16 C, HSQC, HMBC: cyclen-CH₂), 79.6 (C_{q,} 6 C, HSQC, HMBC: C²¹), 96.5 (+, 1 C, HSQC, HMBC, COSY: C⁵H), 108.3 (C_{q,} 1 C, HSQC, HMBC: C⁶), 109.9 (+, 1 C, HSQC, HMBC, COSY: C⁴H), 110.1 (C_{q,} 1 C, HSQC, HMBC: C⁸), 131.1 (+, 1 C, HSQC, HMBC, COSY: C¹¹H), 148.0 (+, 1 C, HSQC, HMBC, COSY: C¹¹H), 152.5 (C_{q,} 1 C, HSQC, HMBC: C³), 156.2 (C_{q,} 6 C, HSQC, HMBC: C²⁰), 157.6 (C_{q,} 1 C, HSQC, HMBC: C¹⁰), 162.5 (C_{q,} 1 C, HSQC, HMBC: C⁹), 163.7 (C_{q,} 1 C, HSQC, HMBC: C¹²), 165.8 (C_{q,} 3 C, HSQC, HMBC: C¹⁷, C¹⁸, C¹⁹).
**IR** (ATR) [cm⁻¹]: ν̃ = 2974, 2933, 1686, 1619, 1584, 1532, 1512, 1466, 1409, 1364, 1246, 1158, 971, 751.
**UV** (CHCl₃): λₘₐₓ (ε) = 419 (41020)
**MS** (ESI(+), DCM/MeOH + 10 mmol/L NH₄Ac): m/z (%) = 1324.0 (100) [MH⁺), 612.5 (12) [M + 2 H⁺]²⁺, 562.5 (25) [M + 2 H⁺ - boc]²⁺, 512.4 (51) [M + 2 H⁺ - 2 boc]²⁺, 462.3 (55) [M + 2 H⁺ - 3 boc]²⁺, 412.3 (34) [M + 2 H⁺ - 4 boc]²⁺, 362.2 (17) [M + 2 H⁺ - 5 boc]²⁺.
*7-Diethylamino-2-oxo-2H-chromene-3-carboxylic acid {2-[4,6-bis-(1,4,7,10-tetraaza-cyclododec-1-yl)-[1,3,5]triazin-2-ylamino]-ethyl}* (**9**):

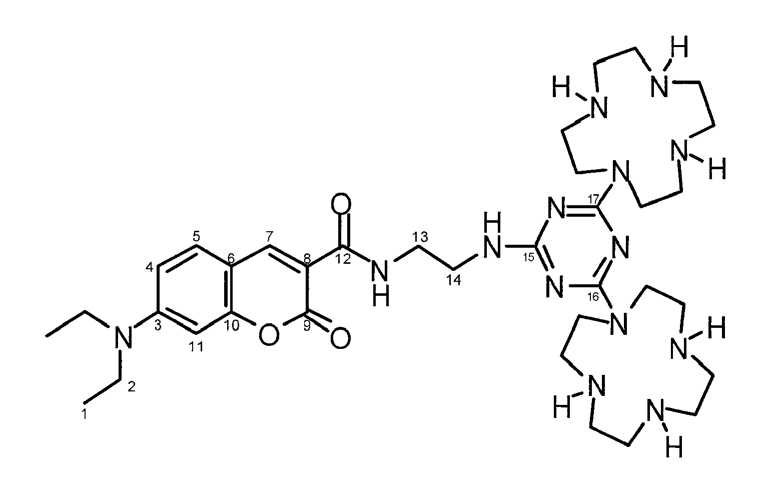

Compound **8** (275 mg, 0.21 mmol) was dissolved in dichloromethane (4 mL) and cooled to 0 °C. Subsequently 4.5 mL of HCl saturated diethylether were added. The solution was stirred 15 min at 0 °C and an additional 20 h at room temperature. The solvent was removed in vacuo, the residue was redissolved in water and extracted with dichloromethane (3x). Subsequently the combined organic layers were dried over MgSO₄ and the solvent was evaporated yielding the protonated hydrochloride of compound 9 as a yellow solid in quantitative yield (213 mg, 0.21 mmol).

**¹H-NMR** (400 MHz; CDCl₃): δ (ppm) = 1.13 (t, ³J = 7.3 Hz, 6 H, HSQC, COSY: C¹H₃), 2.97-3.54 (m, 24 H, HSQC, HMBC, COSY: cyclen-CH₂), 3.57-3.72 (m, 6 H, HSQC, HMBC: C¹³H₂; HSQC, HMBC, COSY: C²H₂), 3.75 (t, ³J = 5.6 Hz, 2 H, HSQC, COSY: C¹⁴H₂), 3.89 (bs, 8 H, HSQC, HMBC, COSY: cyclen-CH₂), 7.37-7.56 (m, 2 H, HSQC, HMBC: C⁴H, HSQC, HMBC: C⁵H), 7.96 (d, ³J = 8.4 Hz, 1 H, HSQC, HMBC: C¹¹H), 8.72 (s, 1 H, HSQC, HMBC: C⁷H).
**¹³C-NMR** (100 MHz; CDCl₃): δ (ppm) = 10.1 (+, 2 C, HSQC, COSY: C¹H₃), 39.0 (-, 1 C, HSQC, HMBC: C¹³H₂), 39.9 (-, 1 C, HSQC, HMBC: C¹⁴H₂), 44.0 (-, 2 C, HSQC, HMBC, COSY: cyclen-CH₂), 44.3 (-, 6 C, HSQC, HMBC, COSY: cyclen-CH₂), 46.1 (-, 4 C, HSQC, HMBC, COSY: cyclen-CH₂), 47.7 (-, 2 C, HSQC, HMBC, COSY: cyclen-CH₂), 48.3 (-, 2 C, HSQC, HMBC, COSY: cyclen-CH₂), 52.6 (-, 2 C, HSQC, HMBC, COSY: C²H₂), 108.8 (+, 1 C, HSQC, HMBC: C⁵H), 117.6 (Cq, 1 C, HSQC, HMBC: C⁶), 117.9 (C_{q,} 1 C, HSQC, HMBC: C⁸), 118.1 (+, 1 C, HSQC, HMBC: C⁴H), 132.5 (+, 1 C, HSQC, HMBC: C¹¹H), 143.2 (C_{q,} 1 C, HSQC, HMBC: C³), 147.6 (+, 1 C, HSQC, HMBC: C⁷H), 154.9 (C_{q,} 1 C, HSQC, HMBC: C¹⁰), 155.2 (C_{q,} 1 C, HSQC, HMBC: C¹⁶), 155.8 (C_{q,} 1 C, HSQC, HMBC: C¹⁵), 161.4 (C_{q,} 1 C, HSQC, HMBC: C⁹), 163.6 (C_{q,} 1 C, HSQC, HMBC: C¹⁷), 164.1 (C_{q,} 1 C, HSQC, HMBC: C⁸).
**MS** (ESI(+), MeCN/TFA): m/z (%) = 362.3 (100) [M + 2 H⁺]²⁺, 723.5 (10) [MH⁺].

To obtain the free base of compound **9** a weakly basic ion exchanger resin was swollen for 15 min in water and washed neutral with water. A column was charged with resin (473 mg, 40.0 mmol hydroxy equivalents at a given capacity of 5 mmol/g). The hydrochloride salt (60 mg, 59 µmol) was dissolved in water, put onto the column and eluated with water. The elution of the product was controlled by pH indicator paper (pH > 10) and was completed when pH was again neutral. The eluate was concentrated and lyophilised to yield 43 mg (59 µmol, 100 %) of free base 9 as a yellow solid.

**MP:** 169 °C
**¹H-NMR** (600 MHz; D₂O): δ (ppm) = 1.04 (t, ³J = 7.1 Hz, 6 H, HSQC, COSY: C¹H₃), 3.02 (bs, 8 H, cyclen-CH₂), 3.12 (bs, 16 H, cyclen-CH₂), 3.27 (q, ³J = 7.0 Hz, 4 H, HSQC, HMBC: C²H₂), 3.38 (t, ³J = 4.6 Hz, 2 H, HSQC, HMBC: C¹³H₂), 3.48 (t, ³J = 5.2 Hz, 2 H, HSQC, HMBC: C¹⁴H₂), 3.62 (bs, 8 H, cyclen-CH₂), 6.13 (d, ⁴J = 1.8 Hz, 1 H, HSQC, HMBC, ROESY: C¹¹H), 6.58 (d, ³J = 9.0 Hz, 1 H, HSQC, HMBC, ROESY: C⁴H), 7.24 (d, ³J = 9.2 Hz, 1 H, HSQC, HMBC, ROESY: C⁵H), 8.09 (s, ⁴J = 1.8 Hz, 1 H, HSQC, HMBC, ROESY: C⁷H), 8.28 (bs, 1 H, HSQC, NH).
**¹³C-NMR** (150 MHz; D₂O): δ (ppm) = 11.8 (+, 2 C, HSQC, HMBC: C¹H₃), 39.4 (-, 1 C, HSQC, HMBC: C¹⁴H₂), 40.1 (-, 1 C, HSQC, HMBC: C¹³H₂), 42.4 (-, 2 C, HSQC, HMBC: cyclen-CH₂), 42.8 (-, 2 C, HSQC, HMBC: cyclen-CH₂), 44.7 (-, 4 C, HSQC, HMBC: cyclen-CH₂), 45.1 (-, 2 C, HSQC, HMBC: C²H₂), 45.7 (-, 4 C, HSQC, HMBC: cyclen-CH₂), 46.1 (-, 4 C, HSQC, HMBC: cyclen-CH₂), 95.5 (+, 1 C, HSQC, HMBC, ROESY: C¹¹H), 106.0 (C_{q,} 1 C, HSQC, HMBC: C⁸), 107.6 (C_{q,} 1 C, HSQC, HMBC: C⁸), 111.4 (+, 1 C, HSQC, HMBC, ROESY: C⁴H), 131.7 (+, 1 C, HSQC, HMBC, ROESY: C⁵H), 148.1 (+, 1 C, HSQC, HMBC, ROESY: C⁷H), 153.6 (C_{q,} 1 C, HSQC, HMBC: C³), 157.0 (C_{q,} 1 C, HSQC, HMBC: C¹⁰), 163.1 (C_{q,} 1 C, HSQC, HMBC: C⁸), 165.3 (C_{q,} 1 C, HSQC, HMBC: C⁹), 166.7 (C_{q,} 1 C, HSQC, HMBC: C¹⁵), 166.8 (C_{q,} 2 C, HSQC, HMBC: C¹⁶, C¹⁷)_{.}
**IR** (ATR) [cm⁻¹]: ν̃ = 3382, 3315, 2968, 2732, 1694, 1576, 1537, 1506, 1417, 1350, 1231, 1134, 1079, 809.

**UV** (CHCl₃): λ (ε) = 260 nm (12474), 426 (43341).
**MS** (ESI(+), TFA/MeCN): m/z (%) = 362.2 (100) [M + 2 H⁺]²⁺, 241.7 (10) [M + 3 H⁺]³⁺, 723.5 (6) [MH⁺], 837.4 (3.0) [MH⁺ + TFA].
**HRMS** Calcd for C₃₅H₅₈N₁₄O₃ 723.4895; Found: 723.4915.

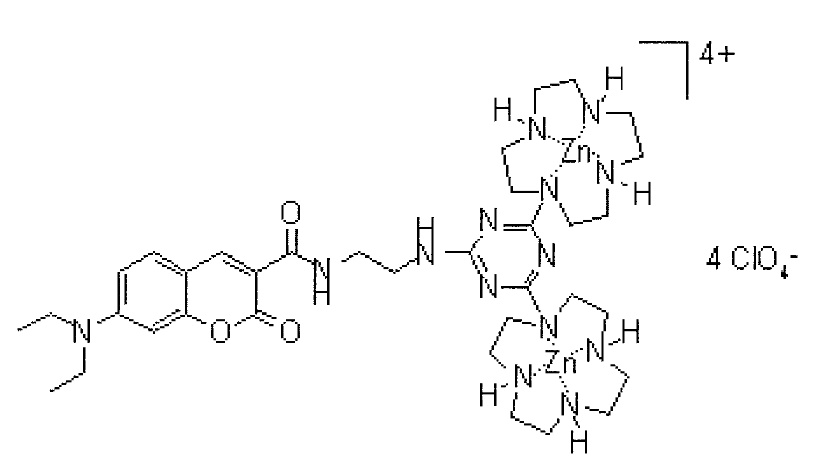

Probe **2**:
Compound **9** (43 mg, 59 µmol) was dissolved in 1 mL of water and heated to 65 °C to get a clear yellow solution. Subsequently zinc(II)-perchlorate (44 mg, 119 µmol) dissolved in 1 ml of water was added slowly. The reaction mixture was stirred for an additional 28 h at 65 °C. The solvent was removed in vacuo and the residue was redissolved in water and lyophilized. Probe **2** (74 mg, 59 µmol, 100 %) was obtained as a yellow solid.

**MP:** 166 °C
**¹H-NMR** (300 MHz; CD₃CN): δ (ppm) =1.19 (t, ³J = 7.0 Hz, 6 H, ethyl-CH₃), 2.45 (bs, 4 H, cyclen-CH₂), 3.02 (bs, 8 H, cyclen-CH₂), 3.26 (bs, 12 H, cyclen-CH₂), 3.37 (bs, 2 H, ethylene diamine-CH₂), 3.49 (q, ³J = 7.0 Hz, 6 H, ethyl-CH₂, ethylene diamine-CH₂), 3.71-4.50 (m, 8 H, cyclen-CH₂), 5.35-6.65 (m, 6 H, cyclen-NH), 6.55 (s, 1 H, CH), 6.80 (d, ³J = 9.1 Hz, 1 H, CH), 7.58 (d, ³J = 9.1 Hz, 1 H, CH), 8.72 (s, 1 H, CH), 9.23 (bs, 1 H, NH).
**IR** (ATR) [cm⁻¹]: ν̃ = 3246, 2938, 2864, 1558, 1457, 1421, 1342, 1287, 1083, 968, 793.
**UV** (HEPES pH 7.4, 25 mM): λ (ε) = 432 (25177).
**MS** (ESI(+), TFA/MeCN): m/z (%) = 485.5 (100) [M⁴⁺ + 2 CH₃COO⁻]²⁺, 455.7 (50) [M⁴⁺ - H⁺ + CH₃COO⁻]²⁺, 425.1 (28) [M⁴⁺ - 2 H⁺]²⁺,

### Example 2: Dephosphorylation by λ-PPase treatment

Bovine α-casein (40 µg, purchased from Sigma-Aldrich) was treated with 400 U of λ-PPase (purchased from New England Biolabs) in Tris-HCl (50 mM), NaCl (100 mM), dithiothreitol (2 mM), MnCl₂ (2 mM), EGTA (0.1 mM), 0.01 % Brij 35, pH 7.5 at 30 °C for 6 h.

### SDS-PAGE

Proteins were resolved on mini gels under denaturating and reducing Laemmli conditions on a PeqLab 45-1010-i apparatus. The gels consisted of a 4 % acrylamide (w/v), 120 mM Tris-HCl (pH 6.8), 0.1 % SDS (w/v) stacking gel and a 15 % acrylamide (w/v), 375 mM Tris-HCl (pH 8.8), 0.1 % SDS (w/v) running gel. A 25 mM Tris, 192 mM glycine, 0.1 % SDS (w/v) running buffer (pH 8.3) was used. Protein samples were heated to 70 °C for 10 min with reducing and denaturating RotiLoad 1 sample buffer (purchased from Carl Roth, Germany) before being loaded onto the gel. The gels were run at 150 V until the proteins entered the running gel, then the voltage was increased to 250 V. Water cooling was used during the entire run. Fixation was accomplished by treating the gels with 50 % MeOH / 10 % AcOH twice, for 30 min and overnight, respectively.

### Staining and Imaging

The gels were soaked in deionized water (4 x 10 min) before being treated with a solution of probe **1** or **2** in deionized water for 1 h with a probe concentration of 10⁻⁷ M. We found destaining was not strictly necessary at this concentration, however, when the probes were used at higher concentrations, the gels could be destained by washing with deionized water until a nonfluorescent background was obtained. Due to their non-covalent binding mode (19), the probes could be completely removed by repeated washing of the gel with water. Conveniently, removal of the probes was not necessary for Coomassie restaining.

The gels were wrapped in cling film to prevent them from drying out and placed on a PeqLab Superbright UV table (λₑₓ = 316 nm). Images were taken using either a Pentax K10D or a Traveler DC 8500. Emission spectra of individual protein bands were obtained using the same UV table and a Hamamatsu PMA-11 photonic multi-channel analyzer. Data were acquired using the supplied PMA Optic software. A 455 nm longpass filter was placed on top of the gel to prevent the UV light saturating the detector. Longpass filters with a shorter cutoff proved unsuitable as they showed a strong fluorescence when subjected to the UV light.

After fluorescence imaging, a restain for total protein was accomplished with 0.1 % Coomassie R-250, 50 % MeOH, 10 % AcOH for 1 h. Destaining was accomplished in 7 % AcOH, 10 % MeOH over night. The gels were again wrapped in cling film and scanned using an office scanner.

### Results and Discussion

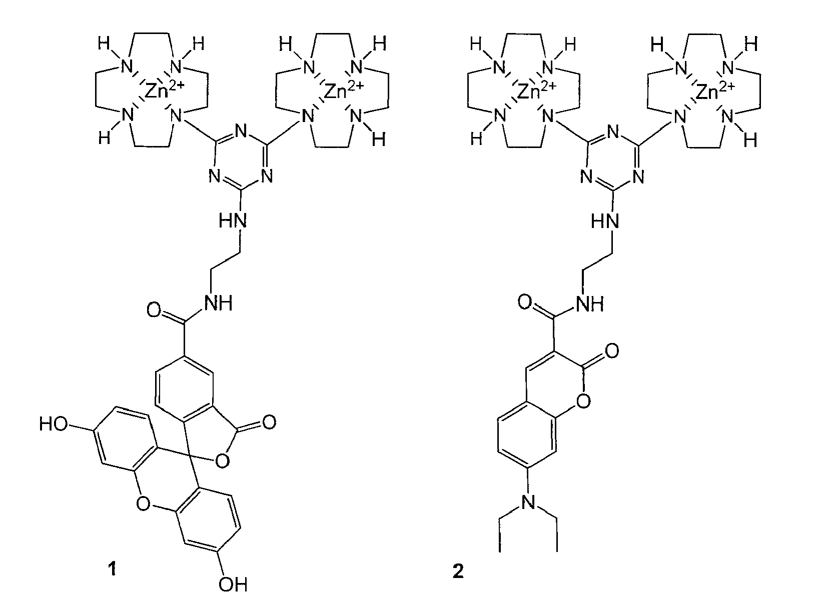

Probes **1** and **2** were used for staining of phosphoproteins (Counterions are not shown).

To evaluate the phospho-staining selectivity and sensitivity of our probes, a dilution series of phosphorylated bovine α-casein was electrophoretically resolved from the non-phosphorylated protein BSA. In addition, a sample of α-casein was dephosphorylated using λ-PPase and used as a control to ensure the emission response would not depend on the amino acid composition of the protein. After fixation, the gels were stained and destained when necessary until little or no background was visible.

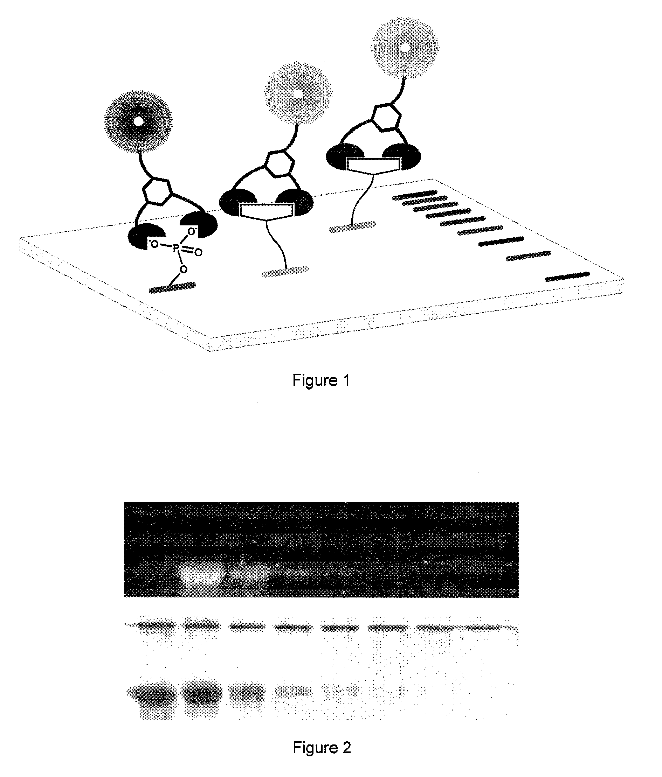

Probe **1** showed a distinct emission in the bands of phosphorylated α-casein, whereas the bands of dephosphorylated α-casein and BSA are barely visible (Figure 2). Bis-zinc(II)-cyclen triazine complexes coordinate phosphate groups strongly, but we also expect an affinity of the probe to non-phosphorylated proteins due to the coordination of histidine by the bis-zinc(II)-cyclen triazine (17, 20) or further unspecific interactions. However, these interactions do not interfere with the specific detection of phosphorylation: The emission of the probe is quenched, when bound to non-phosphorylated amino acid residues and the emission remains, when bound to phosphorylated amino acid residues. Similar emission quenching effects have been previously reported for the interaction of riboflavin with a zinc(II)-imidazole complex (21) and for zinc(II)-porphyrin with histidine (22). To prove that the observed effects originate from the coordination of the bis-zinc(II)-cyclen triazine complex and not from the binding of the fluorophore itself, a control gel was prepared and treated with carboxyfluorescein. No staining could be observed in this experiment.

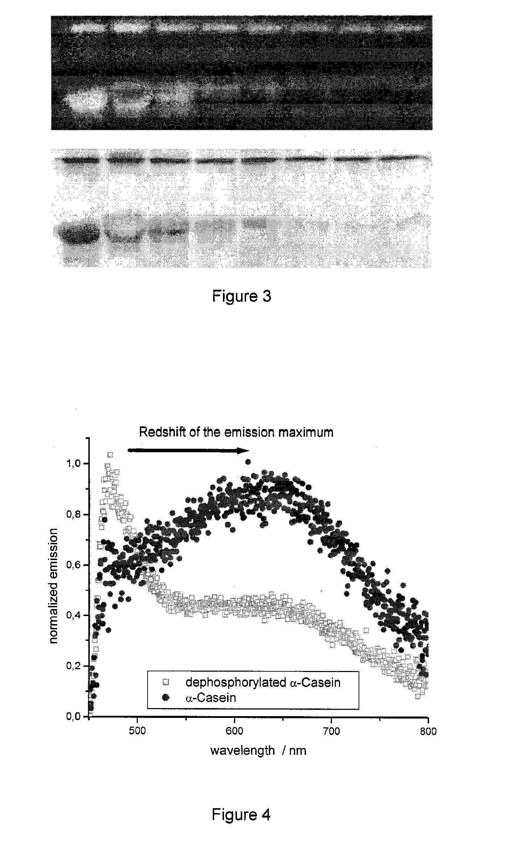

When bound to phosphorylated α-casein, probe **2** showed a strong redshift in the emission compared to unphosphorylated α-casein and BSA (Figure 3). We attribute this spectral change to the different electronic environments when the probe molecule is either unspecifically interacting with non-phosphorylated amino acid residues, such as histidine (unphosphorylated α-casein and BSA) or is coordinating a negatively charged phosphorylated amino acid residue (phosphorylated α-casein). These findings are in agreement with the reported redshift in emission of a mono-zinc(II)-cyclen coumarin complex upon coordination to inorganic phosphate ions (23) To quantify this change in emission, fluorescence spectra of the gel bands were obtained using a photonic multi-channel analyzer equipped with a fiber optic (Figure 4). As with probe **1**, a control gel was treated with the fluorophore itself, and again no staining was observed.

With both probes, the dilution series proved that 62 ng of phosphorylated α-casein are still detectable on a normal UV-table by the unaided eye (which was protected from UV light) while imaging was performed with common digital cameras. Hence, even without the use of specialized equipment like laser-illuminated gel scanners or cooled camera detectors as described in the protocols of commercially available phosphoprotein gel stains our probes reach similar limits of detection.

### Conclusion

The non-covalent, reversible and fluorescent SDS-PAGE probes used in the present examples are capable of indicating protein phosphorylation. The probes show different fluorescence responses discriminating phosphorylated from non-phosphorylated proteins. While probe **1** signals binding to a phosphorylated protein by a significant increase of emission intensity, probe **2** changes its emission spectrum upon binding to a phosphorylated protein. The probes achieve their selectivity through a combination of the specificity of the dinuclear metal chelate binding site towards phosphate oxoanions and a modulation of the chromophore emission due to the proximity of the phosphorylated amino acid. The environment-sensitive fluorophore allows a clear distinction between phosphorylated and non-phosphorylated proteins on SDS-PAGE and allows the detection of 62 ng of phosphorylated α-casein on a normal UV-table.

### Example 3:

### Use of other fluorophores

In order to underline the importance of the properties of the fluorophore in the design of probes 1 and 2, we elucidated the emission response of analogous probes 10 to 12 bearing dansyl, pyrene and Cy3 fluorophores, respectively. The synthesis of compounds 10 to 12 was identical to 1 and 2, apart from attachment of the fluorophore to precursor 3 which can be deduced by a person skilled in the art.

### Synthesis

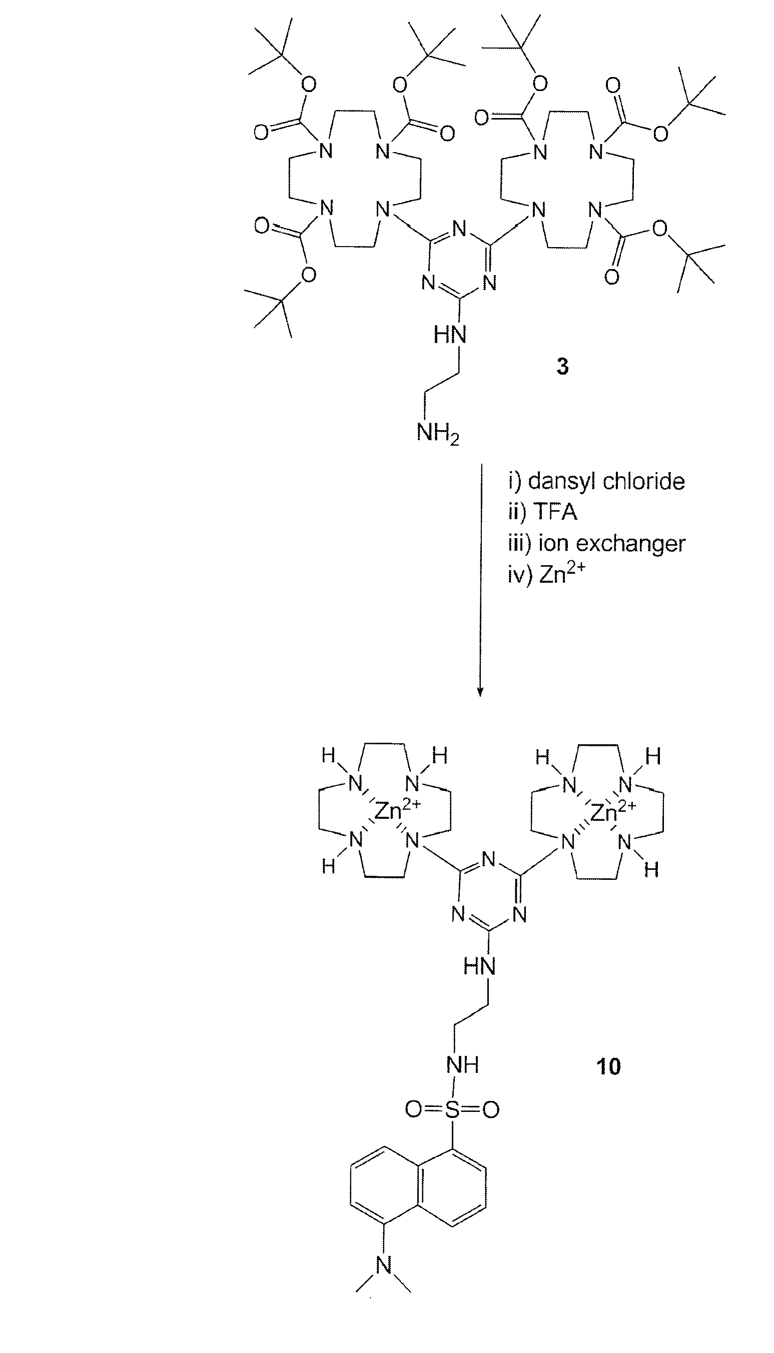

### Synthesis of dansyl-labeled compound 10

In reaction (i), the precursor 3 was reacted with dansyl chloride in DCM at room temperature. After removing the solvent under reduced pressure, treatment with TFA in DCM (1:1) (ii) removed the Boc groups. Deprotonation by a basic ion exchanger (iii) was followed by complexation with Zn(ClO₄)₂ in water at 80 °C (iv). The solvent was removed by lyophilization.

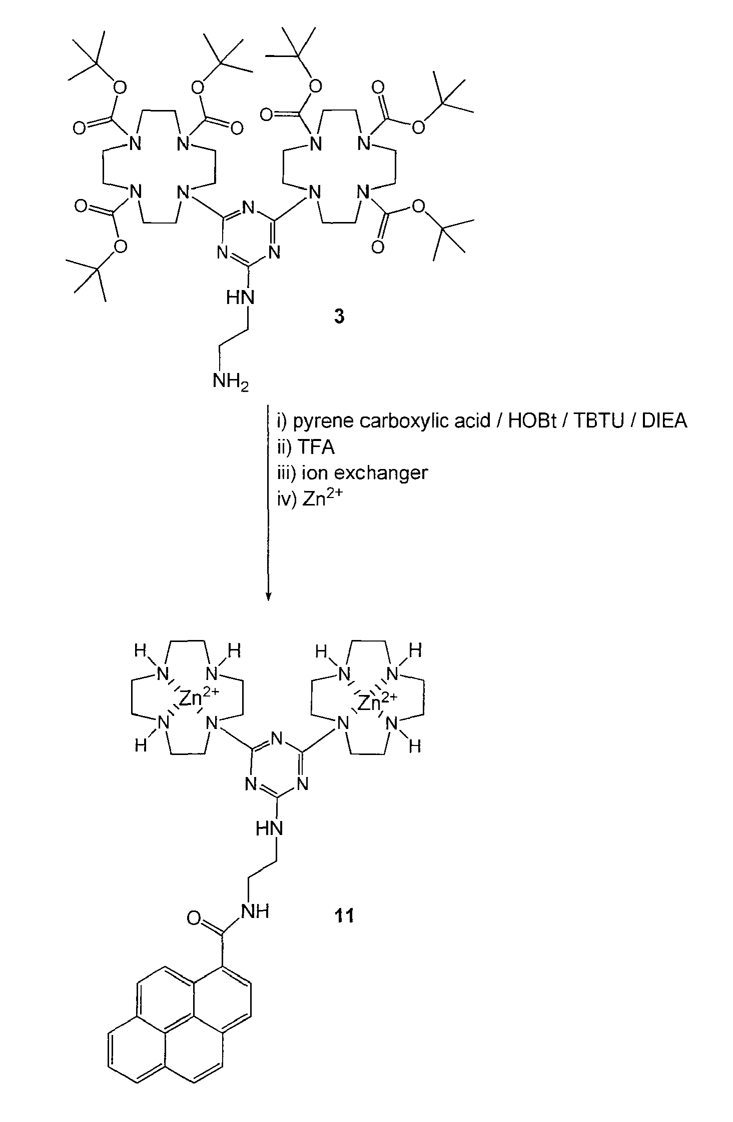

### Synthesis of pyrene-labeled compound 11

In reaction (i), the precursor 3 was reacted with pyrene carboxylic acid in DCM at room temperature using HOBt, TBTU and DIEA as coupling reagents. After removing the solvent under reduced pressure, treatment with TFA in DCM (1:1) (ii) removed the Boc groups. Deprotonation by a basic ion exchanger (iii) was followed by complexation with Zn(ClO₄)₂ in water at 80 °C (iv). The solvent was removed by lyophilization.

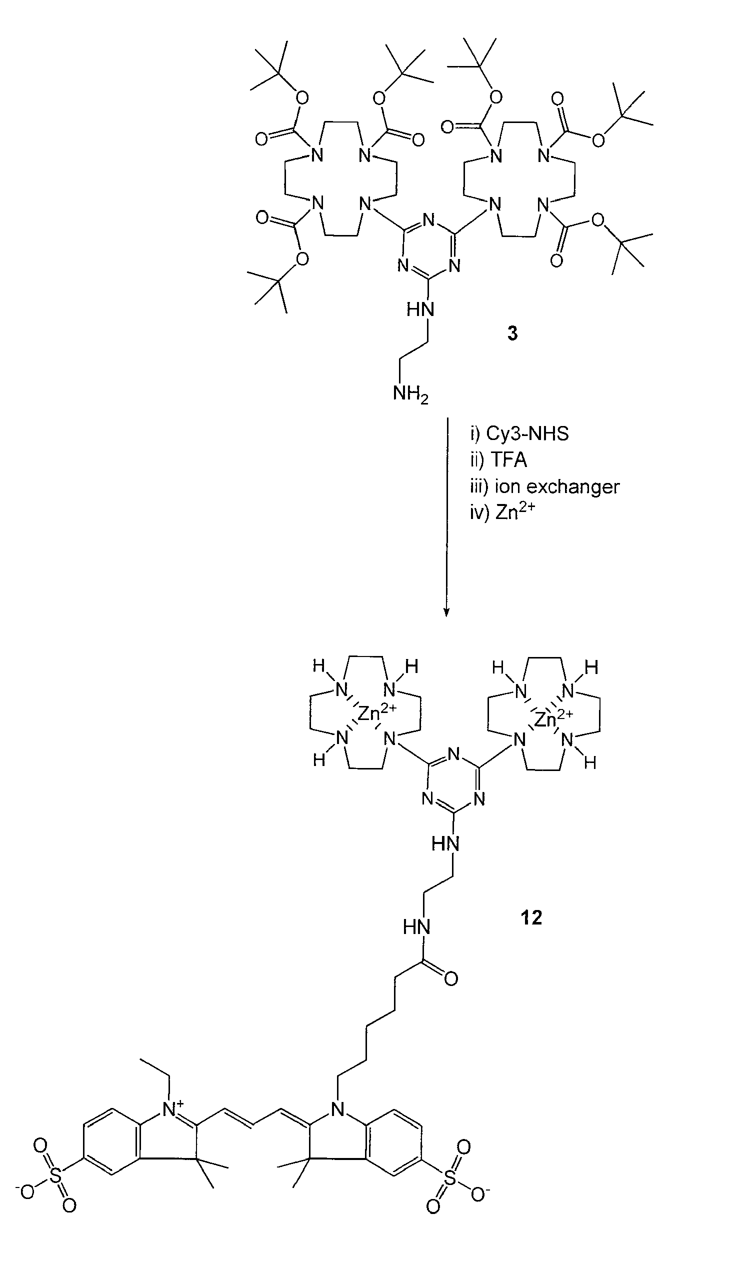

### Synthesis of Cy3-labeled compound 12

In reaction (i), the precursor 3 was reacted with Cy3-NHS in DCM at room temperature. After removing the solvent under reduced pressure, treatment with TFA in DCM (1:1) (ii) removed the Boc groups. Deprotonation by a basic ion exchanger (iii) was followed by complexation with Zn(ClO₄)₂ in water at 80 °C (iv). The solvent was removed by lyophilization.

### Discussion

The labeled metal chelates 10 to 12 were tested for their fluorescence response when bound to phosphorylated and nonphosphorylated protein as described in example 2. None of the compounds allowed a distinction between phosphorylated and non-phosphorylated proteins. The emission did not change either compared to the free probes in solution. These results indicate that the fluorophore is a significant component in the design of probes 1 and 2.

### References

(1) Pío, R., Martinez, A., Unsworth, E. J., Kowalak, J. A., Bengoechea, J. A., Zipfel, P. F., Elsasser, T. H., Cuttitta, F. (2001) Complement Factor H Is a Serum-binding Protein for Adrenomedullin, and the Resulting Complex Modulates the Bioactivities of Both Partners. J. Biol. Chem. 276, 12292-12300.
(2) Misenheimer, T. M., Hahr, A. J., Harms, A. C., Annis, D. S., Mosher, D. F. (2001) Disulfide Connectivity of Recombinant C-terminal Region of Human Thrombospondin 2. J. Biol. Chem. 276, 45882-45887.
(3) Williams, N. K., Prosselkov, P., Liepinsh, E., Line, I., Sharipo, A., Littler, D. R., Curmi, P. M. G., Otting, G., Dixon, N. E. (2002) In Vivo Protein Cyclization Promoted by a Circularly Permuted Synechocystis sp. PCC6803 DnaB Mini-intein. J. Biol. Chem. 277, 7790-7798.
(4) Schulenberg, B., Goodman, T. N., Aggeler, R., Capaldi, R. A., Patton, W. F. (2004) Characterization of dynamic and steady-state protein phosphorylation using a fluorescent phosphoprotein gel stain and mass spectrometry. Electrophoresis 24, 2526-2532.
(5)Kinoshita, E., Kinoshita-Kikuta, E., Takiyama, K., Koike, T. (2006) Phosphate-binding Tag, a New Tool to Visualize Phosphorylated Proteins. Mol. Cell. Proteomics 5, 749-757.
(6) Hunter, T. (1995) Protein kinases and phosphatases: The Yin and Yang of protein phosphorylation and signalling. Cell 80, 225-236.
(7) Hunter, T. (2000) Signaling-2000 and Beyond. Cell 100, 113-127.
(8) Manning, G., Whyte, D. B., Martinez, R., Hunter, T., Sudarsanam, S. (2002) The Protein Kinase Complement of the Human Genome. Science 298, 1912-1934.
(9) Salomon, A. R., Ficarro, S. B., Brill, L. M., Brinker, A., Phung, Q. T., Ericson, C., Sauer, K., Brock, A., Horn, D. M., Schultz, P. G., Peters, E. C. (2003) Profiling of tyrosine phosphorylation pathways in human cells using mass spectrometry. Proc. Natl. Acad. Sci. USA 100, 443-448.
(10) Conrads, T. P., Veenstra, T. D. (2005) An enriched look at tyrosine phosphorylation. Nat. Biotechnol. 23, 36-37.
(11) Larsen, M. R., Sorensen, G. L., Fey, S. J., Larsen, P. M., Roepstorff, P. (2001) Phospho-proteomics: Evaluation of the use of enzymatic de-phosphorylation and differential mass spectrometric peptide mass mapping for site specific phosphorylation assignment in proteins separated by gel electrophoresis. Proteomics 1, 223-238.
(12) Immler, D., Gremm, D., Kirsch, D., Spengler, B., Presek, P., Meyer, H. E. (1998) Identification of phosphorylated proteins from thrombin-activated human platelets isolated by two-dimensional gel electrophoresis by electrospray ionization-tandem mass spectrometry (ESI-MS/MS) and liquid chromatography-electrospray ionization-mass spectrometry (LC-ESI-MS). Electrophoresis 19, 1015-1023.
(13) Ojida, A., Kohira, T., Hamachi, I. (2004) Phosphoprotein-Selective Recognition and Staining in SDS-PAGE by Bis-Zn(II)-dipycolylamine-Appended Anthracene. Chem. Lett. 33, 1024-1025.
(14) Ojida, A., Mito-oka, Y., Inoue, M., Hamachi, I. (2002) First Artificial Receptors and Chemosensors toward Phosphorylated Peptide in Aqueous Solution. J. Am. Chem. Soc. 124, 6256-6258.
(15) Hünig, S., Märkl, G., Sauer, J. (1994) Einführung in die apparativen Methoden in der Organischen Chemie, 2nd Edition, Würzburg, Regensburg.
(16) Schwetlick, K. (2001) Organikum, 21st Edition, Wiley-VCH, Weinheim.
(17) Grauer, A., Riechers, A., Ritter, S., König, B. (2008) Synthetic Receptors for the Differentiation of Phosphorylated Peptides with Nanomolar Affinities. Chem. Eur. J. 14, 8922-8927.
(18) Berthelot, T., Talbot, J.-C., Laïn, G., Déleris, G., Latxague L. (2005) Synthesis of Nε-(7-diethylaminocoumarin-3-carboxyl)- and Nε-(7-methoxycoumarin-3-carboxyl)-L-fmoc lysine as tools for protease cleavage detection by fluorescence. J. Pep. Sci. 11, 153-160.
(19) Kruppa, M., König, B. (2006) Reversible Coordinative Bonds in Molecular Recognition. Chem. Rev. 106, 3520-3560.
(20) Kruppa, M., Frank, D., Leffler-Schuster, H., König, B. (2006) Screening of metal complex-amino acid side chain interactions by potentiometric titration. Inorg. Chim. Acta 359, 1159-1168.
(21) Lugina, L. N., Davidenko, N. K., Gavrish, S. P. (1985) Quenching of the fluorescence of riboflavin by the histidinates and alaninates of nickel, copper, and zinc. Theor. Exp. Chem. 21, 466-470.
(22) Hou, J. L., Yi, H. P., Shao, X.-B., Li, C., Wu, Z.-Q., Jiang, X.-K., Wu, L.-Z., Tung, C.-H., Li, Z.-T. (2005) In Situ Click Chemistry: Enzyme-Generated Inhibitors of Carbonic Anhydrase II. Angew. Chem. 118, 810-814.
(23) Mizukami, S., Nagano, T., Urano, N., Odani, Y., Kikuchi, K. (2002) A Fluorescent Anion Sensor That Works in Neutral Aqueous Solution for Bioanalytical Application. J. Am. Chem. Soc. 124, 3920-3925.

## Claims

1. A compound comprising an anion and a cation having the general formula (I): 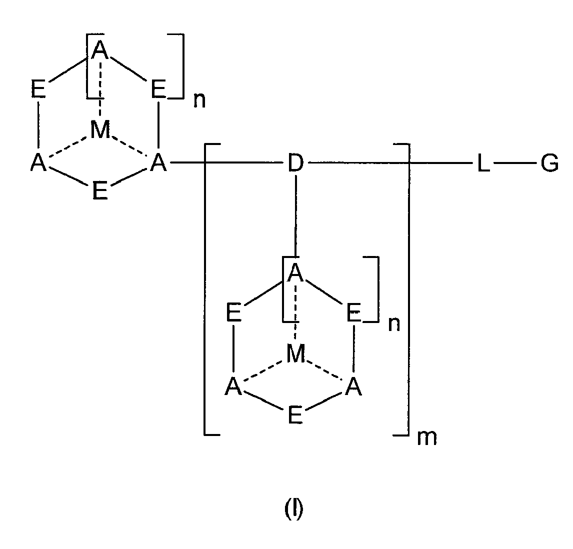 wherein:
A is selected from the group consisting of S, O, NH and PH;
E is selected from the group consisting of CH₂ and CH₂CH₂;
M is selected from the group consisting of Zn, Mn, Fe, Sc, Ti, Zr, Eu, Gd, and Tb;
D is a linking group;
L is a linking group;
G is a substituted or unsubstituted fluorophor group of the general formula (II), (III) or (IV): 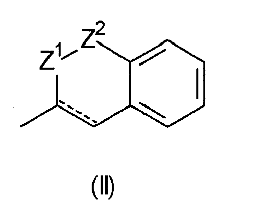 wherein
Z¹ is selected from -CH₂- and -C(O)-;
Z² is selected from -CH₂- and -O-; and
----- is a single or double bond; 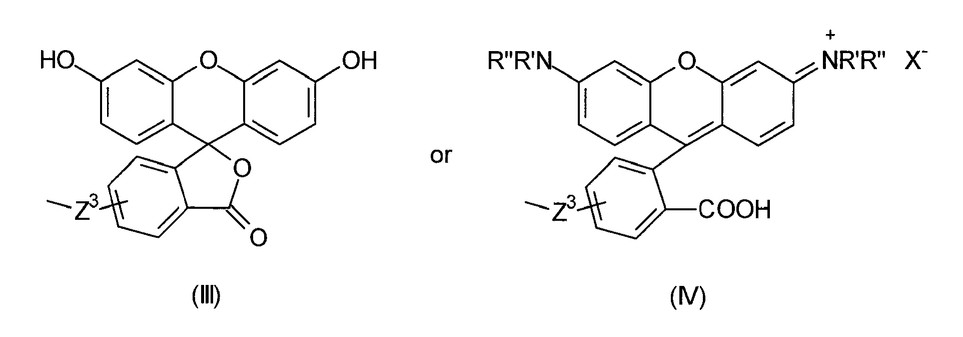 wherein
Z³ is -CH₂-, -C(O)- or -C(S)-;
R' and R" are independently selected for each occurrence and are selected from the
group consisting of H and C₁₋₄ alkyl; and X is an anion;
n is an integer from 1 to 6;
m is 0 or 1, with the proviso that m = 1 if the fluorophor group is substituted or unsubstituted coumarin; and
"-" indicates a chemical bond, while "-----" indicates that the metal M is coordinated by the moiety A.

2. The compound of claim 1, wherein D is

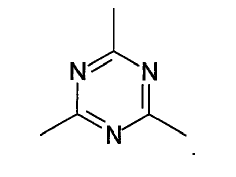

3. The compound of claim 1 or 2, wherein L is -Xₚ-(CH₂)_{q}-Yᵣ- in which X is selected from -O-, -S- and -NH-;
p = 0 or 1;
q = 1 to 6;
Y is selected from -O-, -S-, -NH-, -C(O)-, -SO₂NH-, -NHSO₂-, -C(O)NH-, -NHC(O)-, -C(O)O- and -OC(O)-; and
r is 0 or 1;
wherein the alkylene chain in the group -Xₚ-(CH₂)_{q}-Yᵣ- can optionally be interrupted by 1 or 2 -O-, -S- or -NH- groups with the proviso that the -O-, -S- and -NH-groups, if present, are not directly adjacent to each other or the other heteroatom-containing groups X and Y, if present.

4. The compound of any one of claims 1 to 3, wherein the cation has the formula **1** or **2**:

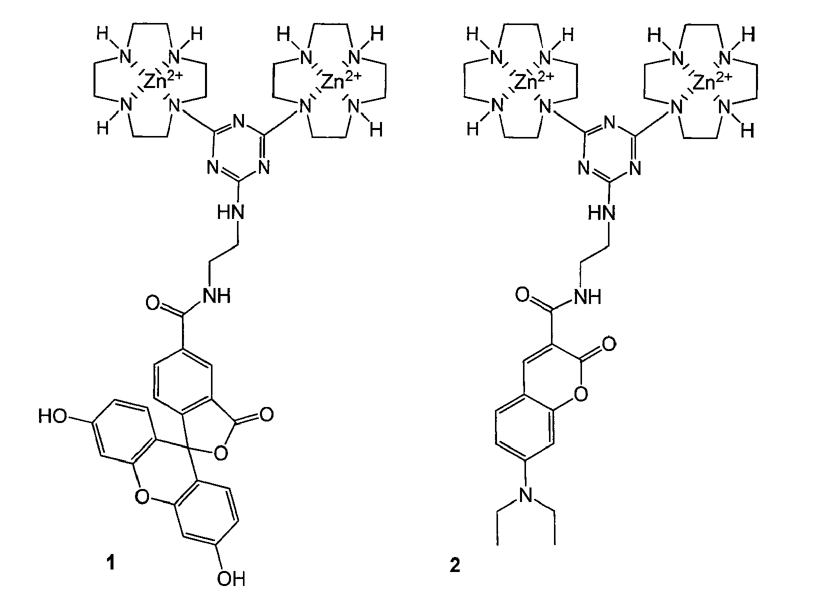

5. A method of detecting a phosphorylated peptide or phosphorylated polypeptide, the method comprising:
(a) bringing into contact said phosphorylated peptide or phosphorylated polypeptide with a compound as defined in any one of claims 1 to 4; and
(b) detecting a complex comprising said phosphorylated peptide or phosphorylated polypeptide and said compound, thereby detecting said phosphorylated peptide or phosphorylated polypeptide.

6. A method of detecting a phosphorylated peptide or phosphorylated polypeptide, the method comprising:
(a) bringing into contact said phosphorylated peptide or phosphorylated polypeptide with a compound comprising an anion and a cation having the general formula (I): 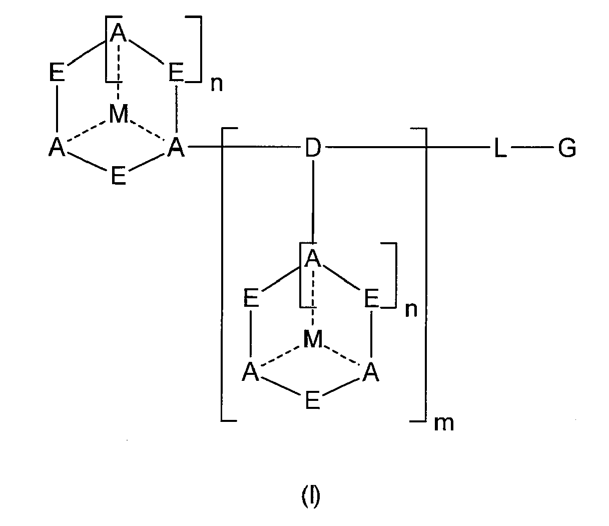 wherein:
A is selected from the group consisting of S, O, NH and PH;
E is selected from the group consisting of CH₂ and CH₂CH₂;
M is selected from the group consisting of Zn, Mn, Fe, Sc, Ti, Zr, Eu, Gd, and Tb;
D is a linking group;
L is a linking group;
G is a substituted or unsubstituted fluorophor group of the general formula (II), (III) or (IV): 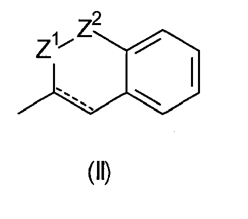 wherein
Z¹ is selected from -CH₂- and -C(O)-;
Z² is selected from -CH₂- and -O-; and
----- is a single or double bond; 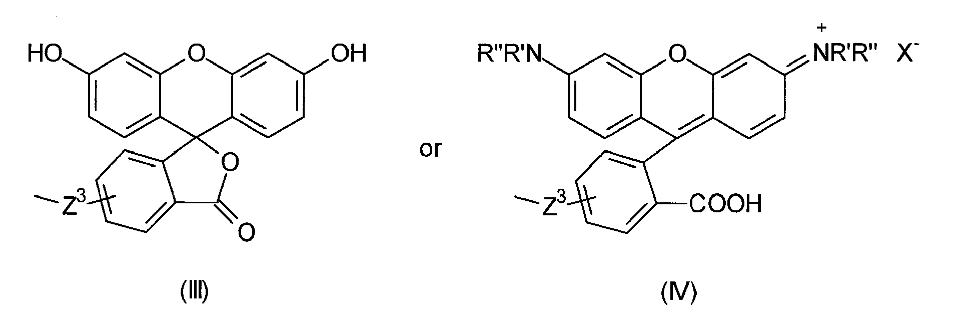 wherein
Z³ is -CH₂-, -C(O)- or -C(S)-;
R' and R" are independently selected for each occurrence and are selected from the group consisting of H and C₁₋₄ alkyl; and
X is an anion;
n is an integer from 1 to 6;
m is 0 or 1; and
"-" indicates a chemical bond, while "-----" indicates that the metal M is coordinated by the moiety A; and
(b) detecting a complex comprising said phosphorylated peptide or phosphorylated polypeptide and said compound, thereby detecting said phosphorylated peptide or phosphorylated polypeptide; wherein the method is effected on or in a support.

7. The method of claim 5 or 6, wherein the detecting of said complex comprises determining whether, upon said bringing into contact,
(i) fluorescence emission of said compound increases, and/or
(ii) the wavelength of the fluorescence emission maximum of said compound is
shifted,
wherein an increase of fluorescence emission and/or shift of the wavelength of the fluorescence emission maximum is indicative of the presence of said complex.

8. A method of detecting and differentiating phosphorylated and unphosphorylated peptides or polypeptides, the method comprising:
(a) bringing into contact said peptides or polypeptides with a compound as defined in any one of claims 1 to 4; and
(b) determining
(i) whether fluorescence emission being characteristic of a complex of said compound with phosphorylated peptides or phosphorylated polypeptides occurs; and
(ii) whether fluorescence emission being characteristic of a complex of said compound with unphosphorylated peptides or unphosphorylated polypeptides occurs,
thereby detecting and differentiating phosphorylated and unphosphorylated peptides or polypeptides.

9. A method of detecting and differentiating phosphorylated and unphosphorylated peptides or polypeptides, the method comprising:
(a) bringing into contact said peptides or polypeptides with a compound as defined in claim 6; and
(b) determining
(i) whether fluorescence emission being characteristic of a complex of said compound with phosphorylated peptides or phosphorylated polypeptides occurs; and
(ii) whether fluorescence emission being characteristic of a complex of said compound with unphosphorylated peptides or unphosphorylated polypeptides occurs,
thereby detecting and differentiating phosphorylated and unphosphorylated peptides or polypeptides;
wherein the method is effected on or in a support.

10. The method of claim 5 or 8, wherein the method is effected on or in a support.

11. The method of claim 6, 9 or 10, wherein said support is selected from
(a) a gel;
(b) a membrane;
(c) a filter; and
(d) an array.

12. The method of claim 11, wherein
(a) said gel is a polyacrylamide gel; or
(b) said membrane is PVDF or nitrocellulose membrane.

13. The method of claim 11 or 12, wherein electrophoresis of said peptide or polypeptide is performed prior to, concomitantly with, or after step (a).

14. The method of claim 5 or 8, wherein the method is effected in solution.

15. Use of a compound as defined in any one of claims 1 to 4 for detecting a phosphorylated peptide or phosphorylated polypeptide.

16. Use of a compound as defined in claim 6 for detecting a phosphorylated peptide or phosphorylated polypeptide, wherein the detection is effected on or in a support.

17. Use of a compound as defined in any one of claims 1 to 4 for detecting and differentiating phosphorylated and unphosphorylated forms of a peptide or polypeptide.

18. Use of a compound as defined in claim 6 for detecting and differentiating phosphorylated and unphosphorylated forms of a peptide or polypeptide, wherein the detection is effected on or in a support.

19. A kit comprising one or more compounds as defined in any one of claims 1 to 4.

20. A diagnostic composition comprising one or more compounds as defined in any one of claims 1 to 4.

21. A method of diagnosing a disease **characterized by** the presence of an aberrant amount of a phosphorylated peptide or phosphorylated polypeptide, the method comprising:
(a) bringing into contact a sample obtained from a subject suspected to suffer from said disease with a compound as defined in any one of claims 1 to 4 or 6; and
(b) determining whether the fluorescence emission of said sample differs from the fluorescence emission of a control sample.
